(19)

Europäisches
Patentamt
European
Patent Office
Office européen
des brevets

(11) **EP 2 626 381 A1**

(12) **EUROPEAN PATENT APPLICATION**
published in accordance with Art. 153(4) EPC

(43) Date of publication:
**14.08.2013 Bulletin 2013/33**

(21) Application number: **11823691.8**

(22) Date of filing: **12.09.2011**

(51) Int Cl.:
*C08J 9/28* (2006.01)   *C08J 3/16* (2006.01)

(86) International application number:
**PCT/JP2011/070757**

(87) International publication number:
**WO 2012/033223 (15.03.2012 Gazette 2012/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.10.2010 JP 2010234060**
**10.09.2010 JP 2010203774**

(71) Applicant: **Kaneka Corporation**
**Osaka-shi, Osaka 530-8288 (JP)**

(72) Inventors:
• **HIRANO, Masaru**
**Takasago-shi**
**Hyogo 676-8688 (JP)**
• **SUZUKI, Yasuyuki**
**Takasago-shi**
**Hyogo 676-8688 (JP)**

• **KANAYA, Kento**
**Takasago-shi**
**Hyogo 676-8688 (JP)**
• **KANDA, Akihisa**
**Takasago-shi**
**Hyogo 676-8688 (JP)**
• **SUZUKI, Takuma**
**Settsu-shi**
**Osaka 566-0072 (JP)**
• **KAWAI, Yoshikazu**
**Takasago-shi**
**Hyogo 676-8688 (JP)**
• **MORIO, Ken-ichiro**
**Takasago-shi**
**Hyogo 676-8688 (JP)**

(74) Representative: **Vossius & Partner**
**Siebertstrasse 4**
**81675 München (DE)**

(54) **METHOD FOR PRODUCING POROUS PARTICLES, POROUS PARTICLES, ADSORBENT BODY, AND METHOD FOR PURIFYING PROTEIN**

(57)     The objective of the present invention is to provide a method for easily producing a porous cellulose particle which can be used as an adsorbent for various substances in a safe manner without using a highly toxic solvent such as a calcium thiocyanate solution. In addition, the objective of the present invention is to provide a porous particle produced by the production method, an adsorbent which contains the porous particle and by which a highly pure protein can be efficiently purified in a safe manner, and a method for purifying a protein by using the adsorbent. The method for producing a porous particle according to the present invention is characterized in comprising the steps of preparing a solution containing cellulose and an ionic liquid; preparing a dispersion by dispersing the obtained cellulose solution into a liquid, wherein the liquid is not compatible with the ionic liquid; and coagulating the dispersion by bringing into contact with an alcohol or an alcohol aqueous solution in order in order to obtain the porous particle.

FIG.6

EP 2 626 381 A1

**Description**

TECHNICAL FIELD

The present invention relates to a method for producing a porous particle, a porous particle produced by the production method, an adsorbent containing the porous particle, and a method for purifying a protein by using the adsorbent.

BACKGROUND ART

A porous particle is widely used as a carrier for an adsorbent such as an adsorbent for various chromatography and an affinity adsorbent. Among such adsorbents, an affinity adsorbent is used as a medical adsorbent and an adsorbent for purifying a medical antibody, since an affinity adsorbent can efficiently purify a target substance and decrease a concentration of an unwanted substance.

In particular, an adsorbent in which protein A is immobilized as an affinity ligand on a porous carrier is at the center of attention as an adsorbent for treating rheumatism, hemophilia and dilated cardiomyopathy (For example, Non-Patent Document 1, Non-Patent Document 2 and Non-Patent Document 3). In addition, an adsorbent in which protein A is immobilized on a porous carrier is also at the center of attention as an adsorbent on which an immunoglobulin, i.e. IgG, can be specifically adsorbed and which is used for purifying a medical antibody.

A polysaccharide is widely used as a material of a medical porous carrier, since a polysaccharide can be industrially produced without difficulty and the safety thereof in a living body is exceedingly high. For example, it is known that a porous cellulose carrier has excellent properties such as a relatively high mechanical strength, a number of hydroxy groups which can be utilized for binding ligands, a low non-specific adsorption and a higher safety in comparison with a synthetic polymer carrier (Patent Documents 1 and 2). For example, Patent Document 3 discloses an adsorbent in which protein A is immobilized on a porous cellulose carrier.

However, a porous cellulose carrier is generally produced by dissolving cellulose in a highly toxic solvent such as a calcium thiocyanate solution, since there are few solvents which can dissolve cellulose. Such a solvent is difficult to be handled in terms of corrosion and safety.

On the other hand, an ionic liquid is recently at the center of attention, since an ionic liquid is non-volatile and a liquid condition can be kept over a wide temperature region. An ionic liquid is mainly applied as a functional solvent, a solvent for an ionic device and a solvent for dissolving a material derived from a living body, such as a polypeptide. It is recently found that an ionic liquid can dissolve cellulose; therefore, an ionic liquid is applied for producing a fiber (Patent Document 4).

PRIOR ART

PATENT DOCUMENT

      Patent Document 1: JP H01-278534 A
      Patent Document 2: JP H11-158202 A
      Patent Document 3: WO 2008/146906
      Patent Document 4: JP 2008-248466 A

NON-PATENT DOCUMENT

      Non-Patent Document 1: Annals of the New York Academy of Sciences, 2005, Vol.1051, pp.635-646
      Non-Patent Document 2: American Heart Journal, Vol.152, No.4, 2006
      Non-Patent Document 3: KASAI Kenichi et al., "Affinity chromatography" published by Tokyo Kagaku Dojin, 1991

DISCLOSURE OF THE INVENTION

PROBLEMS TO BE SOLVED BY THE INVENTION

The objective of the present invention is to provide a method for easily producing a porous cellulose particle which can be used as an adsorbent for various substances in a safe manner without using a highly toxic solvent such as a calcium thiocyanate solution. In addition, the objective of the present invention is to provide a porous particle produced by the production method, an adsorbent which contains the porous particle and by which a highly pure protein can be efficiently purified in a safe manner, and a method for purifying a protein by using the adsorbent.

MEANS FOR SOLVING THE PROBLEMS

The present inventors intensively studied so as to solve the above problems. As a result, the inventors found that an amorphous porous particle which has adequate size and an appropriate porosity as a material of a protein adsorbent can be efficiently produced in a safe manner by dissolving cellulose in an ionic liquid and contacting the obtained solution with a specific solvent, and completed the present invention.

The method for producing a porous particle according to the present invention is characterized in comprising the steps of:

preparing a solution containing cellulose and an ionic liquid;
preparing a dispersion by dispersing the obtained cellulose solution into a liquid, wherein the liquid is not compatible with the ionic liquid; and
coagulating the dispersion by bringing into contact with an alcohol or an alcohol aqueous solution in order to obtain the porous particle.

In the step for preparing the cellulose solution in the present invention method, it is preferred that water and/or an alcohol is added and an amount of the water and/or alcohol to be added is adjusted to be not less than 2 wt% and not more than 20 wt% relative to a total amount with the ionic liquid. In the embodiment, it is possible to control an internal structure of the porous particle. Specifically, it is possible to uniformize a pore diameter of the porous particle by adding a poor solvent of cellulose to the solution.

In the present invention method, it is preferred to adjust a temperature of the cellulose solution to be not less than 0°C and not more than 70°C. When the temperature is not less than 0°C, a solubility of cellulose in an ionic liquid is high and it becomes easy to prepare the dispersion since a viscosity of the cellulose solution is low. In addition, when the temperature is not more than 70°C, the solvent other than the ionic liquid is hardly volatilized when the cellulose solution and the dispersion are prepared.

It is preferred that the dispersion is brought into contact with the alcohol or the alcohol aqueous solution under the condition of not less than 10°C. For reasons that are unknown, the present inventors found that the higher the temperature is when the dispersion is brought into contact with the alcohol or the alcohol aqueous solution, the larger a micropore diameter of the obtained porous particle becomes . Therefore, the temperature is preferably not less than 10°C.

The porous particle according to the present invention is characterized in being produced by the above-described present invention method.

The adsorbent according to the present invention is characterized in comprising an affinity ligand and the porous particle according to the present invention, wherein the affinity ligand is immobilized on the porous particle.

With respect to the porous particle and the adsorbent according to the present invention, it is preferred that a value of k is not more than -0.1 under the condition that the following formula (I) holds when the porous particle or the adsorbent is packed in a column, gel phase distribution coefficients of multiple proteins having equal or higher molecular weight as compared to a target substance to be adsorbed are measured, and measured values are plotted with $K_{av}$ values on an abscissa and logarithm values of the molecular weights on an ordinate:

$$K_{av} = k \times \ln(MW) + b \quad (I)$$

wherein $K_{av}$ represents the gel phase distribution coefficient, MW represents the molecular weight of the protein, and b represents a constant.
When the value of k in the formula (I) is not more than -0.1, an adsorption capacity is becomes more excellent since a specific surface area relative to the molecular weight of the substance to be adsorbed becomes larger.

With respect to the porous particle, it is preferred that the value of $K_{av}$ of the target substance to be adsorbed, specifically IgG, measured by using a column packed with the porous particle is not less than 0.3. When the value of $K_{av}$ is not less than 0.3, an adsorption capacity becomes more excellent since a specific surface area relative to the molecular weight of the substance to be adsorbed becomes larger.

With respect to the porous particle and the adsorbent according to the present invention, it is preferred that an exclusion limit molecular weight is not less than $10^3$ and not more than $10^9$. When the exclusion limit molecular weight is included in the range, various useful proteins can be purified using the porous particle and the adsorbent according to the present invention.

With respect to the adsorbent according to the present invention, it is preferred that an amount of the affinity ligand to be introduced is not less than 1 mg and not more than 1000 mg per mL of the porous particle. When the introduction amount is not less than 1 mL, the adsorbent may have a sufficient adsorption capacity more surely. On the other hand, the introduction amount is preferably not more than 1000 mg, since too much amount affinity ligand may not be possibly introduced onto the carrier and productivity may be possibly decreased in some cases.

An amount of cellulose to be contained in the porous particle and the adsorbent according to the present invention is preferably not less than 2 wt% and not more than 50 wt%. When the content is not less than 2 wt%, the adsorbent which is hardly compressed when purification is carried out in large scale at high velocity can be obtained. In addition, when the content is not more than 50 wt%, sufficient pores through which a target substance to be purified can be passed can be ensured.

It is preferred that the porous particle, which is a carrier of the adsorbent according to the present invention, is cross-linked. The cross-linked porous particle can be used at high linear velocity and under high pressure, since such a particle has a high strength.

With respect to the porous particle and the adsorbent according to the present invention, it is preferred that a compressive stress when the porous particle or the adsorbent is compressed by 10% is not less than 0.01 MPa and not more than 3 MPa. When the compressive stress is not less than 0.01 MPa, the particle and the adsorbent are hardly compressed even when a liquid is passed through at high linear velocity. In addition, when the compressive stress is not more than 3 MPa, brittleness is improved and a microparticulate generation is inhibited more surely.

A volume average particle diameter of the porous particle and the adsorbent according to the present invention is preferably not less than 1 $\mu$m and not more than 2000 $\mu$m. When the volume average particle diameter is not less than 20 $\mu$m, the particle and the adsorbent are hardly compressed more surely. When the volume average particle diameter is not more than 1000 $\mu$m, an amount of a target substance to be adsorbed may be increased.

The affinity ligand of the adsorbent according to the present invention is preferably protein A.

The method for purifying a protein according to the present invention is characterized in comprising the steps of:

packing the above-described adsorbent according to the present invention into a column;
allowing a solution containing a crude protein to pass through the adsorbent packed in the column; and
allowing an eluent to pass through the column.


EFFECT OF THE INVENTION

By the present invention method, a cellulose porous particle can be produced more securely and at lower cost without using a calcium thiocyanate solution and the like, which are conventionally used. In addition, an ionic liquid used in the present invention method, instead of a calcium thiocyanate solution, and a liquid which is not compatible with the ionic liquid can be separated from each other to be recovered after use. The recovered liquids can be reused. Therefore, the present invention method is also suitable for an industrial mass production.

In addition, by the present invention method, hardness and an internal structure of a cellulose porous particle can be improved. In particular, an amorphous cellulose porous particle can be obtained. Therefore, a cellulose porous particle which can be used as a carrier of an adsorbent for various substances can be provided by the present invention method.

Furthermore, in the present invention method, a viscosity of the dispersion obtained from the cellulose solution depends on the liquid which is not compatible with the ionic liquid. Therefore, an apparent viscosity of the cellulose solution having a high concentration can be kept down during handling; as a result, the operatability of the solution can be significantly improved.

In addition, the cellulose porous particle produced by the present invention method is very useful as a carrier of an adsorbent for a protein and the like.


BRIEF DESCRIPTION OF THE DRAWINGS

Figure 1 is a SEM photograph of the surface of the cellulose particle according to the present invention obtained in the Comparative Example 1.
Figure 2 is a photograph which was obtained by highly magnifying the Figure 1.
Figure 3 is a SEM photograph of the torn surface of the cellulose particle according to the present invention obtained in the Comparative Example 1.

Figure 4 is a photograph which was obtained by highly magnifying the Figure 3.

Figure 5 represents XRD patterns of the cellulose particles obtained in the Comparative Examples 1 to 3.

Figure 6 is a SEM photograph of the surface of the cellulose particle according to the present invention obtained in the Example 1.

Figure 7 is a photograph which was obtained by highly magnifying the Figure 6.

Figure 8 is a SEM photograph of the torn surface of the cellulose particle according to the present invention obtained in the Example 1.

Figure 9 is a photograph which was obtained by highly magnifying the Figure 8.

Figure 10 is a SEM photograph of the surface of the cellulose particle according to the present invention obtained in the Example 2.

Figure 11 is a photograph which was obtained by highly magnifying the Figure 10.

Figure 12 is a SEM photograph of the torn surface of the cellulose particle according to the present invention obtained in the Example 2.

Figure 13 is a photograph which was obtained by highly magnifying the Figure 12.

Figure 14 is a SEM photograph of the surface of the cellulose particle according to the present invention obtained in the Example 3.

Figure 15 is a photograph which was obtained by highly magnifying the Figure 14.

Figure 16 is a SEM photograph of the torn surface of the cellulose particle according to the present invention obtained in the Example 3.

Figure 17 is a photograph which was obtained by highly magnifying the Figure 16.

Figure 18 represents XRD patterns of the cellulose particles obtained in the Comparative Examples 1 and Examples 1 to 3.

Figure 19 represents XRD patterns of the cellulose particles obtained in the Examples 3 to 5.

Figure 20 is a SEM photograph of the torn surface of the cellulose particle according to the present invention obtained in the Example 7.

Figure 21 is a photograph which was obtained by highly magnifying the Figure 20.

Figure 22 is a SEM photograph of the surface of the cellulose particle according to the present invention obtained in the Example 8.

Figure 23 is a SEM photograph of the surface of the cellulose particle according to the present invention obtained in the Example 9.

Figure 24 is a SEM photograph of the surface of the cellulose particle according to the present invention obtained in the Example 10.

Figure 25 represents XRD patterns of the cellulose particles obtained in the Examples 8 to 10.

MODE FOR CARRYING OUT THE INVENTION

Hereinafter, the method of the present invention is described in the order depending on the exploitation.

(1) Step for preparing a solution

In the production method of the present invention, first, a solution containing cellulose and an ionic liquid is prepared.

An ionic liquid is generally an ionic compound in the form of a liquid at lower than 100°C under ordinary pressure. A cationic part of an ionic liquid is exemplified by pyridinium, imidazolium, imidazole and the like. An acyclic cation is exemplified by a quaternary alkyl ammonium, a quaternary alkyl phosphonium cation and the like. A counter anion of a cationic part is selected from the group consisting of a halogen ion, a pseudo halogen ion, an organic halide ion which is an organic compound containing a halogen, a carboxylate and the like. A carboxylate is exemplified by an acetate, a citrate, a malate, a maleate, a formate and an oxylate; a halogen ion is exemplified by a chloride, a bromide, a zinc chloride / a choline chloride; and an organic halide ion is exemplified by a tetrafluorobarate ion, a hexafluorophosphate ion, $CF_3SO_2^-$, $(CF_3SO_2)_2N^-$, $CF_3CO_2^-$, 3-methyl-N-butyl-pyridinium chloride and benzyldimethyl(tetradecy)ammonium chloride.

Any cellulose which can be dissolved in an ionic liquid may be used in the present invention. For example, one or more kinds of celluloses selected from a regenerated cellulose, a crystalline cellulose or a microcrystalline cellulose, and cellulose acetate may be used. In particular, a crystalline cellulose having an average polymerization degree of not less than about 100 and not more than about 700 is preferred. When the average polymerization degree is not less than 100, the strength of the porous particles to be obtained can be made sufficient. On the other hand, when the average polymerization degree may be too high, the viscosity of the cellulose solution can be excessively high and therefore, the average polymerization degree is preferably not more than 700. The average polymerization degree is more preferably not less than 100 and not more than 500, furthermore preferably not less than 150 and not more than 350, and particularly preferably not less than 200 and not more than 300. In particular, a crystalline cellulose having an average polymerization degree of about 300 is preferred. In the case of using a crystalline cellulose with a high average polymerization degree, it is effective to add a poor solvent or to carry out operation at a high temperature for lowering the viscosity of the cellulose solution.

An amount of cellulose to be used is not particularly limited and may be properly adjusted. For example, such an amount is preferably not less than 2 wt% and not more than 50 wt% relative to 100 wt% of the cellulose solution. When the concentration is not less than 2 wt%, an adsorbent which is not compacted even if purification is carried out at a high linear rate or in a large scale may be obtained. On the other hand, when the concentration is not more than 50 wt%, a sufficient pore through which a target substance to be purified can pass can be secured. In addition, when the concentration is too high, the viscosity of the solution may be so excessively high as to possibly worsen the operability and therefore, the concentration is preferably not more than 50 wt%. The concentration is more preferably not less than 3 wt%, furthermore preferably not less than 4 wt%, particularly preferably not less than 5 wt%, and more preferably not more than 30 wt%, furthermore preferably not more than 20 wt%, particularly preferably not more than 15 wt%.

A method for preparing the solution is not particularly limited and a common method may be employed. For example, the solution may be prepared by simply mixing cellulose with an ionic liquid and heating or stirring the mixture until the cellulose is sufficiently dissolved.

An inner structure of the porous particle can be controlled by adding one or more kinds selected from water and/or an alcohol to the solution containing cellulose and an ionic liquid to an extent that cellulose is not precipitated. More specifically, a pore diameter of the porous particle can be made more uniform by adding a poor solvent for cellulose to the solution.

In the case where an alcohol aqueous solution is added, an alcohol concentration may be properly adjusted; and for example, a ratio of water : an alcohol may be set as [80 : 20] to [5 : 95] by volume.

An amount of water and/or an alcohol to be added may be properly adjusted to an extent that cellulose is not precipitated at ordinary temperature and for example, the amount may be adjusted to be not less than 2 wt% and not more than 20 wt% relative to the total amount of an ionic liquid and water and/or an alcohol to be added. When the additive amount is not less than 2 wt%, an effect to make a pore diameter of the porous particle uniform is more reliably exerted. On the other hand, when the additive amount is too high, cellulose may be possibly precipitated and therefore, the additive amount is preferably not more than 20 wt%, and more preferably not more than 15 wt%.

At the time of dissolving cellulose in an ionic liquid, the mixture may be heated. However, since a common ionic liquid is in a form of a liquid at lower than 100°C, it is preferred to keep the temperature lower than 100°C. The temperature is more preferably not less than 50°C and not more than 90°C. In order to carry out the dissolution easily, cellulose may be previously microparticulated.

(2) Dispersion step

Next, the obtained cellulose solution is dispersed in a liquid which is not compatible with the ionic liquid to prepare a dispersion.

A temperature of the above-described cellulose solution is preferably adjusted to be not less than 0°C and not more than 70°C before or during dispersion in a liquid which is not compatible with the ionic liquid. When the temperature is not less than 0°C, a solubility of cellulose in the ionic liquid is high, and the viscosity of the cellulose solution is lowered so as to make the preparation of the dispersion easy. When the temperature is not more than 70°C, a solvent other than the ionic liquid is hardly evaporated at the time of preparation of the cellulose solution or the dispersion.

It is preferred that not less than 1000 mL of a liquid which is not compatible with an ionic liquid is needed for dissolving 1 g or 1 mL of the employed ionic liquid at 25°C. Such a liquid may be selected depending on the kind of an ionic liquid to be used, and is exemplified by hexane, ethyl acetate, a $C_{6-12}$ linear saturated fatty acid, a $C_{16-24}$ unsaturated fatty acid, a fat and oil which is derived from an animal or a plant and of which melting point is not more than 100°C, a hydrogenated oil derived from an animal or a plant, a fractionated oil obtained by fractionizing and purifying a high melting point fraction of a fat and oil derived from an animal or a plant or a hydrogenated product thereof, a saturated fatty acid triglyceride, an edible wax, a microalgae-derived fat and oil, a microorganism fat and oil, a medium chain fatty acid triglyceride, an unsaturated fatty acid triglyceride, and the like. The fat and oil to be used for the present invention is not particularly limited as long as the fat and oil has a melting point of not more than 100°C. The fat and oil is exemplified by a fat and oil derived from an animal or a plant, such as a palm oil, a shea butter, a sal butter, an illipe butter, a lard, a tallow, a rapeseed oil, a rice oil, a peanut oil, an olive oil, a corn oil, a soybean oil, a perilla foil, a cotton seed oil, a sunflower oil, an evening primrose oil, a sesame oil, a safflower oil, a coconut oil, a cocoa butter, a palm kernel oil and a fish oil; a hydrogenated animal or plant oil such as a hydrogenated palm oil, an extremely hydrogenated palm oil, a hydrogenated rapeseed oil, an extremely hydrogenated rapeseed oil, a hydrogenated soybean oil, a hydrogenated lard oil and a hydrogenated fish oil; a fractionated oil obtained by fractionizing and purifying high melting point fraction of a fat and oil derived from an animal or a plant or a hydrogenated product thereof, such as a fractionated coconut oil, a fractionated palm kernel oil, a fractionated palm oil, a fractionated cocoa butter oil, a fractionated shea butter oil, a fractionated lard oil, a fractionated oil of a hydrogenated soybean oil, and a fractionated oil of a hydrogenated fish oil; a saturated fatty acid triglyceride, such as tristearin, tripalmitin and trilaurin; a partial glyceride of a fatty acid, such as a monoglyceride thereof and a diglyceride thereof; an edible wax such as a bees wax, a candelilla wax and a rice bran wax; a seaweed-derived fat and oil, such as an Undaria oil and a Laminaria oil; a microalgae-derived fat and oil, such as a Spirulina-extracted oil; a microorganism fat and oil, such as a yeast-extracted oil and a Mortierella-extracted oil; a fractionated oil obtained by fractionizing and purifying a low melting point fraction of the above-described oils and fats, such as a low melting point fraction of a palm oil, a low melting point fraction of a fish oil, and a low melting point fraction of a shea butter; a medium chain fatty acid triglyceride such as tricaprylin and tricaprin; an unsaturated fatty acid triglyceride such as triolein and trilinolein; a partial glyceride such as a monoglyceride thereof and a diglyceride thereof; and a fatty acid such as oleic acid, linoleic acid, linolenic acid, arachidonic acid, eicosapentaenoic acid and docosahexaenoic acid.

Only one kind of the above-described liquid which is not compatible with an ionic liquid may be used alone, or two or more kinds may be used in the form of a mixture. The examples of the case of mixing two or more kinds may include a mixed solvent of saturated fatty acids with different numbers of carbon atoms, a mixed solvent of unsaturated fatty acids with different numbers of carbon atoms, and a mixed solvent of a saturated fatty acid and an unsaturated fatty acid.

A surfactant may be used for dispersing an ionic liquid in a liquid which is not compatible with the ionic liquid. Such a surfactant is exemplified by N-acyl-L-glutamic acid triethanolamine, sodium N-acyl-L-glutamate, an alkyldiaminoethylglycine hydrochloride liquid, dimethicone copolyol, polyoxyethylene octyl phenyl ether, polyoxyethylene stearyl ether, polyoxyethylene cetyl ether, polyoxyethylene-polyoxypropylene glycol, glyceryl monostearate, propylene glycol monostearate, polyoxyethylene glyceryl monostearate, polyoxyethylene sorbitan monostearate, polyethylene glycol monophosphate and the like. From the viewpoints of coloration suppression on cellulose, solution viscosity and the like, a medium chain fatty acid triglyceride is preferred as a surfactant.

Droplets of the cellulose solution are formed by dispersing the cellulose solution in the above-described liquid. The use amounts of the cellulose solution and a liquid which is not compatible with the ionic liquid are not particularly limited and may be properly adjusted, and a ratio of the liquid : the cellulose solution may be adjusted to be [98 : 2] to [50 : 50] by volume. The volume ratio is preferably [97 : 3] to [70 : 30], and more preferably [97 : 3] to [80 : 20].

A method for dispersing the cellulose solution is not particularly limited, and for example, the cellulose solution can be homogeneously dispersed in a liquid which is not compatible with the ionic liquid by stirring using a stirring blade or a homogenizer. A static mixer can also be employed.

(3) Solidification step

Next, the above-mentioned dispersion is solidified by bringing into contact with an alcohol or an alcohol aqueous solution to obtain porous particles. In the present step, an ionic liquid in the droplets is extracted to insolubilize and condense cellulose and thus porous particles consisting of cellulose can be obtained.

An alcohol to be employed in the present step is exemplified by a $C_{1-6}$ alcohol such as methanol, ethanol, 1-propanol, 2-propanol, n-butanol, isobutanol, 2-butanol, sec-butanol, 2-methyl-2-propanol (tert-butanol), 1-pentanol, 2-pentanol, 3-pentanol, 2-methyl-1-butanol, 3-methyl-1-butanol, 2-methyl-2-butanol, 3-methyl-2-butanol, 1-hexanol, 2-hexanol, 3-hexanol and the like.

A concentration of an alcohol aqueous solution is not also particularly limited and may be properly adjusted, and for example, a ratio of water : an alcohol may be adjusted to be [80 : 20] to [5 : 95] by volume. Surprisingly, the inventors of the present invention found that fine pores of the porous particle become larger as the volume ratio of water in the alcohol aqueous solution is higher, and that the sphericity of the porous particles becomes higher and the porous particles become more amorphous as the volume ratio of alcohol is higher. From the viewpoints, the volume ratio is more preferably [70 : 30] to [10 : 90], furthermore preferably [50 : 50] to [10 : 90], and particularly preferably [40 : 60] to [20 : 80].

A method for bringing the above-described dispersion into contact with an alcohol or an alcohol aqueous solution is not particularly limited, and the entire amount of the alcohol or the alcohol aqueous solution may be added to the dispersion, a portion of the alcohol or the alcohol aqueous solution may be added step by step to the dispersion, the entire amount of the dispersion may be added to the alcohol or the alcohol aqueous solution, or a portion of the dispersion may be added step by step to the alcohol or the alcohol aqueous solution. Preferably, the dispersion is gradually added to a stirred alcohol or alcohol aqueous solution.

An amount of an alcohol or an alcohol aqueous solution to be used is not particularly limited and may be a sufficient amount to extract the ionic liquid, and for example, the volume ratio of the alcohol or alcohol aqueous solution relative to the dispersion may be adjusted to be not less than 1 and not more than 5.

A temperature at the time of the contact is not also particularly limited and is preferably adjusted to be not less than the melting point of the ionic liquid and not more than the boiling point of an alcohol to be used. When the temperature is adjusted to not more than the melting point of the ionic liquid, it may possibly become difficult to release the ionic liquid from the cellulose particles. On the other hand, when the temperature is adjusted to not less than the boiling point of the alcohol, it results in increased costs since to a refluxing apparatus or the like is necessary. The temperature is preferably not less than 10°C and not more than 60°C, more preferably not less than 25°C and not more than 60°C, and particularly preferably not less than 40°C and not more than 55°C. Additionally, a porosity of the solidified cellulose particle is surprisingly affected by the temperature of the cellulose solution and either the alcohol or the alcohol aqueous solution, and a fine pore diameter of the cellulose particle becomes larger as the solution temperature is higher. Also in the point, the temperature is preferably adjusted within the above-described preferred range.

With respect to a fine pore diameter of the porous cellulose particle, for example, the maximum diameter thereof is preferably not less than 200 angstroms and not more than 1500 angstroms, and the average diameter thereof is preferably not less than 100 angstroms and not more than 400 angstroms. The average diameter is more preferably not less than 140 angstroms and not more than 400 angstroms, and furthermore preferably not less than 200 angstroms and not more than 400 angstroms.

The cellulose particle obtained in the above-described manner is porous, and the size of the pore and the hardness thereof can be controlled by changing a cellulose concentration, a dispersion temperature, and a cellulose-insoluble liquid.

(4) Recovery step

Next, the obtained porous particle may be recovered. A means for recovery is not particularly limited, and a common solid-liquid separation means such as filtration and centrifugation may be employed.

The obtained porous particle may be washed with water or an alcohol and thereafter dried in reduced pressure in order to remove the ionic liquid and the cellulose-insoluble liquid remaining in the pores if necessary.

The used ionic liquid can be recovered by evaporating the cellulose-insoluble liquid from the obtained liquid by solid-liquid separation.

(5) Crosslinking step

The porous particle obtained by the above-described method of the present invention can be further improved in strength by a crosslinking agent. Such crosslinking agent and crosslinking reaction condition are not particularly limited, and known techniques can be employed. The crosslinking agent is exemplified by a halohydrine such as epichlorohydrin, epibromohydrin and dichlorohydrin; a bifunctional bisepoxide (bisoxirane); and a polyfunctional polyepoxide (polyoxirane).

The size of the porous particle is not particularly limited, and in general, is preferably not less than about 1 $\mu$m and not more than about 2 mm. For example, when the porous particle is used as an adsorbent carrier for purification, a volume average particle diameter is preferably not less than 20 $\mu$m and not more than 1000 $\mu$m. When the volume average particle diameter of the porous particle is not less than 20 $\mu$m, compaction is difficult to be caused. When the volume average particle diameter is not more than 1000 $\mu$m, an adsorption amount of a target substance to be purified is increased in the case where the porous particles are used for an adsorbent carrier for purification. The volume average particle diameter of the porous particle is more preferably not less than 20 $\mu$m, furthermore preferably not less than 30 $\mu$m, particularly preferably not less than 50 $\mu$m, most preferably not less than 60 $\mu$m, and more preferably not more than 500 $\mu$m, furthermore preferably not more than 250 $\mu$m, particularly preferably not more than 125 $\mu$m, most preferably not more than 95 $\mu$m. The volume average particle diameter can be determined by measuring the particle diameter of randomly selected 100 porous particles. The particle diameter of each porous particle can be measured by photographing a microscopic photograph, of each porous particle, saving the photograph in the form of electronic data, and carrying out an analysis using a particle diameter measurement software such as Image-Pro Plus manufactured by Media Cybernetics, Inc. Alternatively, the measurement can also be carried out by using a laser diffraction/scattering particle size distribution measuring device. The size of the porous particle can be controlled by the stirring intensity, the kind of a surfactant or the like at the time of the dispersion or at the time of bringing the dispersion into contact with an alcohol or an alcohol aqueous solution.

Next, a method for producing an adsorbent is described. In the method, the cellulose porous particle obtained in the above-described manner is used as a carrier.

(6) Step for immobilizing affinity ligand

In order to produce an adsorbent to adsorb a protein, an affinity ligand which has high affinity to a target substance to be adsorbed is immobilized on the above-described porous cellulose particle.

**EP 2 626 381 A1**

An affinity ligand means a molecule or a compound which is interacted with a specific target substance and is capable of having a specific affinity to the specific target substance. For example, when an antibody is a target substance to be adsorbed, an affinity ligand is exemplified by an antigen and a protein which cause specific interaction, a peptide having antibody bonding activity, and the like.

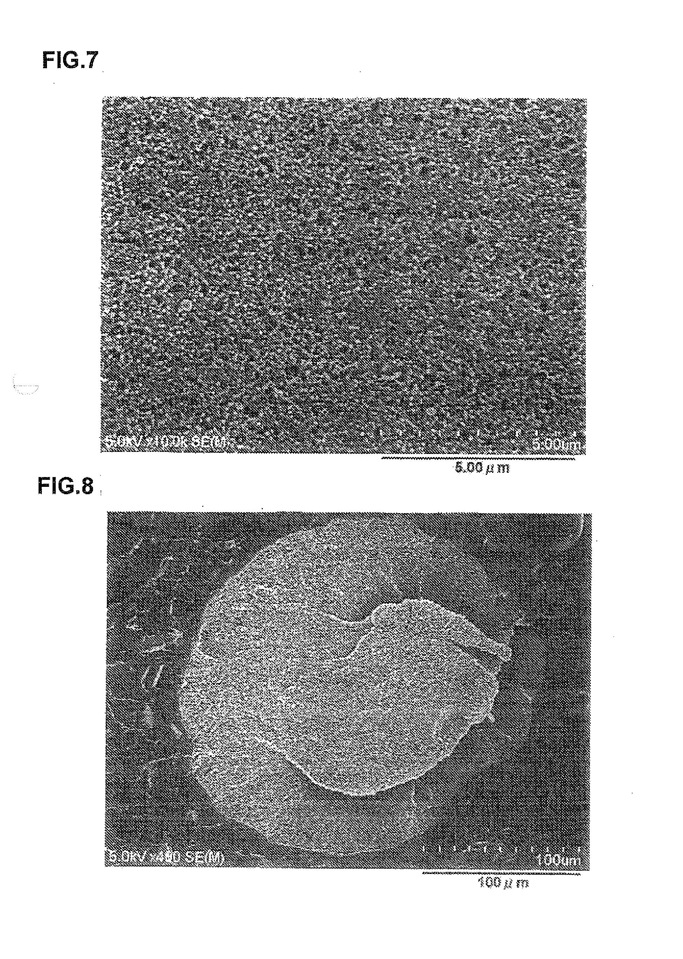

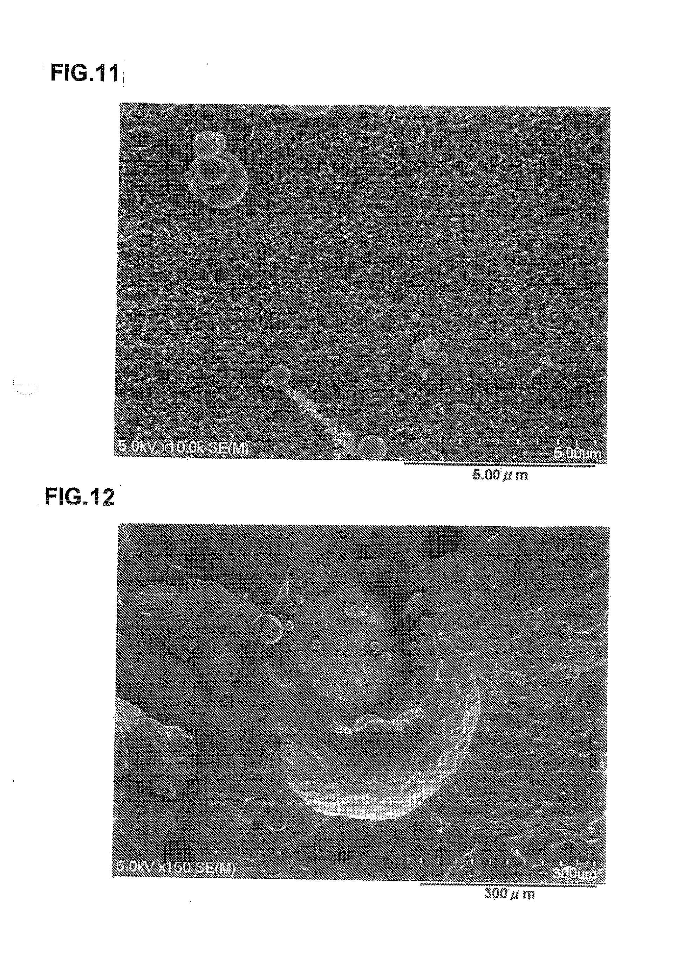

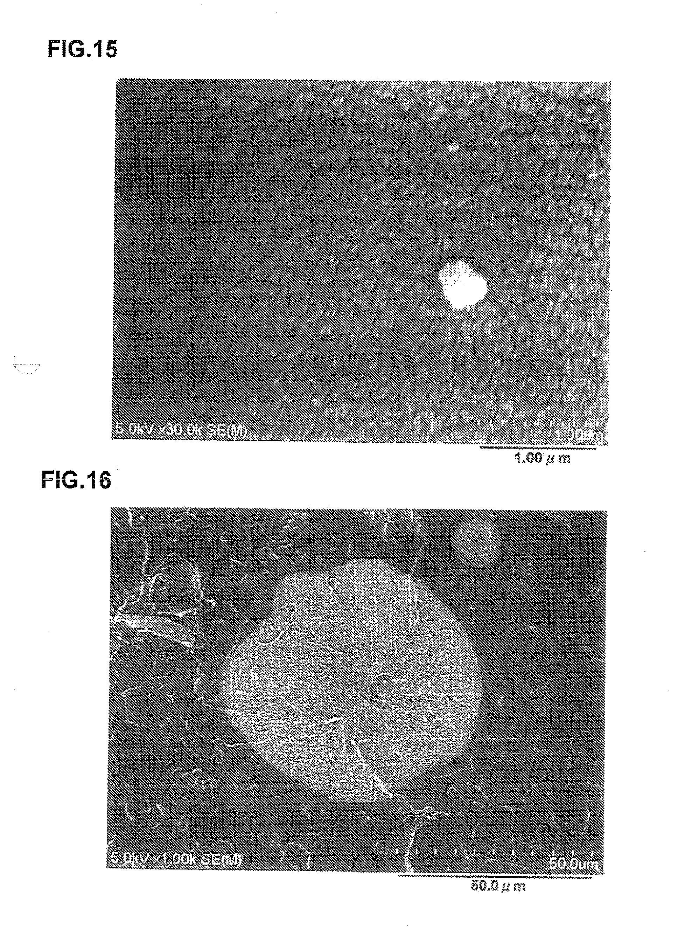

An affinity ligand to be used for the adsorbent of the present invention is not particularly limited, and various affinity ligands can be immobilized by using a porous carrier which is activated for immobilizing a desired affinity ligand. A method of immobilization is exemplified by a method for immobilizing an amino group-containing ligand by employing a cyanogen bromide method, a trichlorotriazine method, an epoxy method, a tresyl chloride method, a periodate oxidation method, a divinylsulfonic acid method, a benzoquinone method, a carbonyl diimidazole method, an acyl azide method and the like; a method for immobilizing a hydroxy group-containing ligand by employing an epoxy method, a diazo coupling method and the like; a method for immobilizing a thiol group-containing ligand by employing an epoxy method, a tresyl chloride method, a divinylsulfonic acid method and the like; a method for immobilizing a carboxylic acid-containing ligand or a formyl group-containing ligand on an aminated carrier; and the like, which are described in Table 8-1, Table 8-2 and Fig. 8-15 of Non-Patent Document 3.

The adsorbent of the present invention can be used as an adsorbent for purification and can also be used as an adsorbent for purifying an antibody pharmaceutical product, which has drawn attention in recent years. An affinity ligand to be used for purifying an antibody pharmaceutical product is not particularly limited, and is exemplified by an antigen and a protein which have high specificity to an antibody, protein G, protein L, a variant thereof, and an amino group-containing ligand such as a peptide having an antibody bonding activity.

In particular, as an adsorbent which can specifically adsorb and elute an immunoglobulin (particularly, IgG), an adsorbent obtained by immobilizing protein A as an affinity ligand on a porous carrier is drawing attention. The protein A to be used for the present invention is not particularly limited, and a natural protein A, a gene recombinant protein A and the like can be used without limitation. In addition, protein A may include an antibody bonding domain and a variant thereof, and may be a fusion protein and the like. Furthermore, it is also possible to use protein A which is produced from a microbe extract or a culture supernatant by combining and/or repeating purification methods selected from a molecular weight fraction method and fractional precipitation method which employs various kinds of chromatography such as ion exchange chromatography, hydrophobic interaction chromatography, gel filtration chromatography and hydroxyapatite chromatography, and a membrane separation technique. In particular, protein A obtained by the method disclosed in WO 2006/004067, US 5151350 B, WO 2003/080655, JP 2006-304633 A or Japanese Patent Application No. 2009-071766 is preferred.

A method for introducing protein A as an affinity ligand into the porous particle can be selected from the various immobilization methods as described above, and a method of immobilization by utilizing the reaction of a formyl group which is possessed by the porous particle and the amino group of protein A is more preferred. For example, a method described in Patent Document 1 can be used.

An amount of an affinity ligand to be introduced into the adsorbent of the present invention is not particularly limited, and for example, may be adjusted to be not less than 1 mg and not more than 1000 mg per 1 mL of the porous carrier. When the ratio is not less than 1 mg, the adsorption amount for a target substance to be purified may be preferably increased. When the ratio is not more than 1000 mg, a production cost may be preferably suppressed. The amount of the affinity ligand to be introduced is more preferably not less than 2 mg, furthermore preferably not less than 4 mg, particularly preferably not less than 5 mg, and more preferably not more than 500 mg, furthermore preferably not more than 250 mg, particularly preferably not more than 200 mg, most preferably not more than 100 mg per 1 mL of the porous carrier.

Hereinafter, preferred physical properties of the porous particle in the case where the porous particle is used as a porous carrier for the adsorbent is described.

When the porous particle is used for the adsorbent, a fine pore diameter thereof is one of important factors. For example, when a fine pore diameter is smaller than a target substance to be adsorbed, adsorption is caused only on the surface of the adsorbent carrier and an adsorption amount may possibly become low. On the other hand, when a fine pore diameter is too large, a specific surface area becomes small and an adsorption amount may possibly become low. Accordingly, the porous carrier which has a proper fine pore diameter for a target substance to be adsorbed is preferred.

One of the physical properties with respect to a fine pore diameter of the porous particle is $K_{av}$. $K_{av}$ means a gel phase distribution coefficient, and is a variable number which is independent from a column and which is calculated in accordance with the following formula from an elution or retention volume $V_R$ (expressed also as $V_e$) for molecules in a prescribed size, a void volume $V_o$ and a geometrical volume $V_c$ of a column.

$$K_{av} = (V_R - V_o) / (V_t - V_o)$$

(for example, refer to "Handbook of Process Chromatography, A Guide to Optimization, Scale-Up and validation" (1997) Academic Press, San Diego. Gail Sofer & Lars Hagel eds. ISBNO-12-654266-X, p. 368)

The reason for using $K_{av}$ value but not an accurate value of a fine pore diameter is because it is difficult to accurately measure the fine pore diameter when the porous particles are dispersed in a solvent and made into a wet state like a gel for column chromatography, and an estimated fine pore diameter measured in a dry state cannot accurately reflect a fine pore diameter in a wet state.

An adsorption amount can be improved by immobilizing an affinity ligand on the porous carrier which shows a straight line with an inclination of not more than -0.1 in a range of molecular weight of equal to or more than the molecular weight of a target substance to be adsorbed when $K_{av}$ values are plotted on the abscissa and the logarithm values of the molecular weights are plotted on the ordinate. In other words, an adsorbent obtained by immobilizing an affinity ligand on the porous carrier of which value of k is not more than -0.1 (k = -0.1 or k < -0.1) under the condition that the following formula (I) holds in a molecular weight region of equal to or more than the molecular weight of a target substance to be adsorbed.

$$K_{av} = k \times \ln(MW) + b \quad (I)$$

wherein k represents an inclination, MW represents a molecular weight of a marker when $K_{av}$ is measured, and b represents a constant.

When a value of k is smaller in the formula (I), a specific surface area for a molecular weight of a substance to be adsorbed becomes larger, and thus an adsorption amount can be increased. Accordingly, a value of k is preferably not more than -0.15, more preferably not more than -0.20, furthermore preferably not more than -0.30, and particularly preferably not less than -5.0 and not more than -0.35.

It is preferred that a value of $K_{av}$ for a molecular weight of a target substance to be adsorbed is not less than 0.3. When a value of $K_{av}$ for a molecular weight of a target substance to be adsorbed is not less than 0.3, a specific surface area for the molecular weight of the substance to be adsorbed becomes larger, and efficient adsorption is made possible. A value of $K_{av}$ is more preferably not less than 0.55, furthermore preferably not less than 0.65, and particularly preferably not less than 0.75. In addition, a value of $K_{av}$ is preferably not more than 0.94. When a value of $K_{av}$ is more than 0.94, a resin content is lowered and a strength of the adsorbent may possibly be lowered.

It is preferred that an exclusion limit molecular weight of the porous carrier is higher than a molecular weight of a target substance to be adsorbed. However, when a fine porous diameter is large and an exclusion limit molecular weight is rather much larger than a molecular weight of a target substance to be adsorbed, adsorption efficiency may be lowered. For example, when the adsorbent is used for purification, a target substance to be adsorbed is often a protein and generally, a molecular weight of a protein is not less than about $10^3$ and not more than about $10^8$ and therefore, it is preferred that an exclusion limit molecular weight is set to be not less than about $10^3$ and not more than about $10^9$. For example, when IgG is a target substance to be adsorbed, it is preferred that an exclusion limit molecular weight is set to be not less than about $10^6$ and not more than about $10^8$.

An exclusion limit molecular weight can be determined by employing a conventionally known method. For example, an exclusion limit molecular weight is a molecular weight when $K_{av}$ is 0, and therefore can be determined according to the following formula (II) obtained by substituting 0 for $K_{av}$ in the above-described formula (I).

$$\text{Exclusion limit molecular weight} = \exp(-b/k) \quad (II)$$

In order to accurately measure physical properties relevant to a fine pore diameter, such as $K_{av}$ and an exclusion limit molecular weight, it is preferred to carry out the measurement by using a compound which is not adsorbed to the adsorbent, and it is more preferred to carry out the measurement by using the porous particle before immobilization of a ligand. When the measurement is carried out using the adsorbent, it is necessary to carry out the measurement using a compound which has a similar molecular weight to a target substance to be adsorbed, or to predict the result by carrying out the measurement using compounds which have molecular weights smaller and higher than that of a target substance to be adsorbed and forming a straight line between two points obtained by the measurement. A marker compound to be used for the measurement is preferably a compound which is analogous with a target substance to be adsorbed. For example, when a synthetic polymer such as polystyrene is compared with a protein, even if the molecular weights thereof are same, the volumes thereof are different in a measurement solution. Accordingly, for example, when a target substance to be adsorbed is an antibody, it is preferred to use a protein as a marker compound.

According to the above-described method of the present invention, a porous particle which has high cellulose content can be produced, since a cellulose solution with high concentration can be prepared by using an ionic liquid. Such a porous particle cannot be obtained by a conventional method. In general, when resin content is high, an adsorbent which does not cause compaction can be obtained. Cellulose content per packing volume of the present invention adsorbent is not particularly limited, and is preferably not less than 2 wt% and not more than 50 wt%. When cellulose content per packing volume is not less than 2 wt%, it is possible to preferably obtain an adsorbent which does not cause compaction even if purification is carried out at a high linear rate or in a large scale. On the other hand, when cellulose content per pecking volume is not more than 50 wt%, sufficient pores which are preferably capable of passing a target substance to be purified can be secured. A cellulose content per packing volume is more preferably not less than 3 wt%, furthermore preferably not less than 4 wt%, and more preferably not more than 25 wt%, furthermore preferably not more than 15 wt% . Cellulose content per packing volume can be determined according to the following formula (III) as follows. That is, the porous particles are packed by precipitation until a volume of the porous particles is not further decreased, and a porous particle amount is adjusted so that the porous particle volume is 1 mL. The particles are dried at 105°C for 12 hours, and the dry weight percent per 1 mL of gel, that is, the cellulose content, can be determined from a dry weight (g) per packing volume.

$$\text{Cellulose content (\%) = [dry weight (g) / packing volume (mL)]} \times 100 \text{ (III)}$$

It is preferred that a compressive stress when the adsorbent of the present invention is compressed by 5% is not less than 0.005 MPa and not more than 1 MPa, a compressive stress when the adsorbent is compressed by 10% is not less than 0.01 MPa and not more than 3 MPa, and a compressive stress when the adsorbent is compressed by 15% is not less than 0.03 MPa and not more than 5 MPa. If a compressive stress when the adsorbent is compressed by 5% is not less than 0.005 MPa and a compressive stress when compressed by 10% is not less than 0.01 MPa and a compressive stress when compressed by 15% is not less than 0.03 MPa, it is easy to obtain an adsorbent which does not cause compaction even if a liquid is introduced at a high linear rate. If a compressive stress when the adsorbent is compressed by 5% is not more than 1 MPa and a compressive stress when compressed by 10% is not more than 3 MPa and a compressive stress when compressed by 15% is not more than 5 MPa, a brittleness is preferably improved and fine particle generation can be preferably suppressed. In the present invention, a compressive stress when compressed by 5% means a stress at the time of 5% decrease in volume from the initial volume when the adsorbent is compressed; a compressive stress when compressed by 10% means a stress at the time of 10% decrease in volume from the initial volume when the adsorbent is compressed; and a compressive stress when compressed by 15% means a stress at the time of 15% decrease in volume from the initial volume when the adsorbent is compressed. The initial volume is a volume in a state where the adsorbent is deposited to be packed until a volume of the adsorbent is not further decreased while applying vibration to a slurry containing the adsorbent. A compressive stress when the adsorbent is compressed can be measured by the following method.

1) The adsorbent is added with water in an amount to allow the entire volume to be 2 times to produce a slurry, and the slurry is loaded into a graduated cylinder made of glass having an inner diameter of 15 mm.

2) The adsorbent is deposited to be packed until a volume of the adsorbent is not further decreased by applying vibration to the graduated cylinder made of glass, and an amount of the adsorbent is adjusted so that a volume of the adsorbent is about 4 mL. The volume at the time is defined as the initial volume.

3) A piston made of a metal which does not cause friction with the inner wall of the graduated cylinder and which is processed to prevent elution of the adsorbent is attached to an autograph (EZ-TEST, manufactured by SHIMADZU CORPORATION) equipped with a load cell for 20N.

4) The position of the piston is adjusted so that the bottom surface of the piston is moved to the position corresponding to 120 vol% of the adsorbent.

5) The piston is lowered at a testing rate of 5 mm/min in a manner of preventing entrainment of bubbles, and the porous carrier is compressed and the volume is decreased.

6) The compressive stress at a prescribed point is measured.

The adsorbent obtained by immobilizing an affinity ligand on the porous carrier having the above-described physical properties is expected to efficiently adsorb a target substance to be adsorbed at a high linear rate and in a large scale.

The adsorbent of the present invention is preferably has an adsorption amount of a target substance to be adsorbed of not less than 1 mg per 1 mL of the adsorbent. When the adsorption amount of a target substance to be adsorbed is not less than 1 mg per 1 mL of the adsorbent, purification can be preferably carried out efficiently. On the other hand, when the adsorption amount of a target substance to be adsorbed is not more than 1000 mg per 1 mL of the adsorbent, an adsorbed target substance can be easily eluted from the adsorbent. The adsorption amount of a target substance to be adsorbed by the adsorbent is more preferably not less than 5 mg, furthermore preferably not less than 10 mg, particularly preferably not less than 20 mg, most preferably not less than 30 mg, and preferably not more than 500 mg, furthermore preferably not more than 300 mg, particularly preferably not more than 250 mg, most preferably not more than 200 mg per 1 mL of the adsorbent.

An amount of a target substance to be adsorbed can be determined as follows. A method for determining an adsorption amount of a target substance is not particularly limited, and an adsorption amount can be determined as a static adsorption amount or as a dynamic adsorption amount. For example, a static adsorption amount can be determined by bringing a solution obtained by dissolving 70 mg of a target substance to be adsorbed in 35 mL of a phosphate buffer (produced by Sigma) having a pH of 7.4 into contact with 0.5 mL of an adsorbent of which solvent is substituted with a phosphate buffer (produced by Sigma) having a pH of 7.4, stirring the mixture at 25°C for 2 hours, and then measuring a decreased amount of the target substance to be adsorbed in a supernatant.

Further, one example of a method for determining a dynamic adsorption amount of a target substance to be adsorbed and a concentration of a ligand which is leaked in the target substance is described as follows.

(1) Preparation of Solution

The following solutions are prepared:

A solution: a phosphate buffer which has a pH of 7.4 (produced by Sigma);
B solution: 35 mM sodium acetate which has a pH of 3.5 (prepared from acetic acid and sodium acetate produced by Wako Pure Chemical Industries, Ltd. and RO water);
C solution: 1 M acetic acid (prepared from acetic acid produced by Wako Pure Chemical Industries, Ltd. and RO water);
D solution: a 1 mg/mL human polyclonal IgG solution (prepared from Gammagard produced by Baxter and the A solution);
E solution: 6 M urea;
F solution: a liquid obtained by adding 0.2 vol% of a surfactant (polyoxyethylene (20) sorbitan monolaurate, produced by Wako Pure Chemical Industries, Ltd.) to the A solution;
Neutralizing solution: 2M tris(hydroxymethyl)aminomethane (prepared from tris(hydroxymethyl)aminomethane produced by Sigma and RO water).

The respective solutions are defoamed before use.

(2) Packing and preparation

As a column chromatography apparatus, AKTA explorer 100 (manufactured by GE Healthcare Bioscience) is used. A mesh of 22 μm is attached to a column having a diameter of 0.5 cm and a height of 15 cm. Into the column, 3 mL of each adsorbent of the present invention is loaded and packed by passing a 20% ethanol aqueous solution (prepared from ethanol produced by Wako Pure Chemical Industries, Ltd. and RO water) at a linear rate of 400 cm/hr for 1 hour. Sampling tubes having a volume of 15 mL are set to a fraction collector, and sampling tubes for an effluent are previously filled with the neutralizing solution.

(3) Washing

If necessary, the respective solutions in an amount of 3 times as much as that of the adsorbent are allowed to pass at a linear rate of 300 cm/hr in the order of the F solution, the B solution, the A solution, the C solution and the E solution. The solution passing cycle is repeated an arbitrary number of times.

(4) IgG purification

The A solution in an amount of 9 mL is allowed to pass at a linear rate of 300 cm/hr and subsequently, while monitoring UV, the D solution is allowed to pass at a linear rate of 300 cm/hr until 10% of IgG is eluted. Next, 30 mL of the A solution is allowed to pass at a linear rate of 300 cm/hr and 30 mL of the B solution is allowed to pass at a linear rate of 300 cm/hr to elute IgG. Subsequently, 9 mL of the C solution is allowed to pass at linear rate of 300 cm/hr and 9 mL of the E solution is allowed to pass at linear rate of 300 cm/hr. The operation after completion of the packing of the adsorbent is repeated further 2 times to measure the IgG amount in the effluent and the concentration of the ligand which leaks in IgG.

The adsorbent of the present invention can be used for purifying various kinds of target substances such as a protein by employing affinity chromatography, for various kinds of purification method as disclosed in Non-Patent Document 3, and for an adsorbent of therapy and an adsorbent for medical treatment. A purification method or a therapeutic method is not particularly limited, and the methods disclosed in Non-Patent Documents 1, 2 and 3 and other known methods can be preferably applied.

In the purification method in which the adsorbent for purification according to the present invention is used, it is preferred that a column having a diameter of not less than 0.5 cm and a height of not less than 3 cm is used. When the diameter is not less than 0.5 cm and the height is not less than 3 cm, purification can be carried out efficiently. From the viewpoint of an accuracy and efficiency of purification, a size of the column is preferably not more than 2000 cm in diameter and not more than 5000 cm in height. A size of a column is more preferably not less than 2 cm and not more than 400 cm in diameter and not less than 5 cm and not more than 400 cm in height, furthermore preferably not less than 5 cm and not more than 300 cm in diameter and not less than 8 cm and not more than 200 cm in height, particularly preferably not less than 10 cm and not more than 250 cm in diameter and not less than 12 cm and not more than 100 cm in height, and most preferably not less than 20 cm and not more than 150 cm in diameter and not less than 15 cm and not more than 50 cm in height.

Therapy or purification in which the adsorbent of the present invention is used preferably comprises the step of allowing a liquid to pass at a linear rate of not less than 10 cm/hr. When the step of allowing a liquid to pass at a linear rate of not less than 10 cm/hr is involved, therapy or purification can be carried out efficiently. From the viewpoints of an accuracy of therapy or purification and a durability of apparatus, therapy or purification using the adsorbent of the present invention is preferably carried out at a linear rate of not more than 5000 cm/hr. The linear rate for purification is more preferably not less than 50 cm/hr, furthermore preferably not less than 100 cm/hr, particularly preferably not less than 200 cm/hr, most preferably not less than 300 cm/hr, and more preferably not more than 3000 cm/hr, furthermore preferably not more than 1500 cm/hr, particularly preferably not more than 1000 cm/hr, most preferably not more than 750 cm/hr.

EXAMPLES

Hereinafter, examples of the present invention are described; however, the present invention should not be limited to the examples.

A tapped volume is used for representing a volume of the porous particle which was used for a reaction when the adsorbent according to the present invention was produced, unless otherwise specified. A tapped volume is a volume of the porous particle under the condition that a slurry of the porous particle and RO water is added into a measuring container and the porous particles are deposited until the volume is not decreased any more while applying vibration. A volume of the porous particle as a base of a ratio of a functional group is also a tapped volume unless otherwise specified. In the case where the porous particle was deficient in respective tests, the porous particle was produced in the same manner to be added.

First, the measurement conditions for physical properties of the respective porous particles are described.

Test Example 1: Measurement of formyl group content

Porous particles (4 mL) of which solvent was substituted with a 0.1 M phosphate buffer having a pH of 8 were brought into contact with 2mL of a 0.1 M phosphate buffer solution having a pH of 8 in which phenylhydrazine was dissolved, and the mixture was stirred at 40°C for 1 hour. Next, the maximum absorbance of the supernatant of the reaction mixture around 278 nm was measured by UV absorbance measurement. From the measurement result, the formyl group content was estimated as the adsorption amount of phenylhydrazine to the porous particles. At the time, the loading amount of phenylhydrazine was adjusted to be 3 times by mole as much as that of the expected formyl group content. When the adsorption amount to the porous particles was not more than 15% or not less than 45% relative to the loading amount of phenylhydrazine, the loading amount of phenylhydrazine was reexamined and the measurement was to be carried out again.

Test Example 2: Measurement of gel phase distribution coefficient (K$_{av}$)

(1) Packing into column

Porous particles were dispersed in RO water and degassed for 1 hour. The degassed porous particles were packed in a column (Tricorn 10/300, manufactured by GE Healthcare Japan) at a linear rate of 105 cm/hr. Thereafter, an eluent (129 mL) having a pH of 7.5 was allowed to pass through the column at a linear rate of 26 cm/hr.

(2) Addition of marker

The followings were used as a marker.
Blue Dextran 2000 (manufactured by Pharmacia Fine Chemicals)

Low Density Lipoprotein (manufactured by Sigma), MW 3,000,000

Thyroglobulin (manufactured by Sigma), MW 660,000

Ferritin (manufactured by Sigma), MW 440,000

· Aldolase (manufactured by Sigma), MW 158,000

· Human IgG (manufactured by Sigma), MW 115, 000

· Bovine Serum Albumin (manufactured by Wako), MW 6,700

· Cytochrome C (manufactured by Wako), MW 12,400

· Bacitracin (manufactured by Wako), MW 1, 400

The above-described markers were diluted with a buffer having a pH of 7.5 so that the concentrations were adjusted to be 5 mg/mL. While the above-described eluent was allowed to pass through the column at a linear rate of 26 cm/hr, each 12 μL of the diluted solutions were injected. The concentration of each marker was finely adjusted as necessary.

(3) Measurement

As a measurement apparatus, DGU-20A3, SCL-10A, SPD-10A, LC-10AD, SIL-20AC, CTO-10AC (all of which were manufactured by SHIMADZU CORPORATION) were used, and LC Solution was used as measurement software. For a liquid volume measurement, a 50 mL-graduated cylinder was used.
Simultaneously with the marker injection, monitoring with UV and measurement of the liquid volume were started in accordance with the following conditions.

1) A liquid volume corresponding to the first peak for Blue Dextran was defined as V$_0$ (mL) .

2) A liquid volume corresponding to the peak for each marker was defined as V$_R$ (mL).
3) A total volume of the porous particles in the column was defined as V$_t$ (mL).
(4) Calculation
A gel phase distribution coefficient (K$_{av}$) of each marker was calculated according to the following formula.
$$K_{av} = (V_R - V_o) \ / \ (V_t - V_o)$$

(5) Maximum diameter
The values of K$_{av}$ and logarithm of the molecular weight of each marker were plotted, and the inclination and the intercept of the following formula were determined from the portion showing linearity.

$$K_{av} = k \times \ln(MW) + b \quad (I)$$

Subsequently, from the measured inclination and the intercept, the molecular weight when a value of $K_{av}$ is 0, that is, an exclusion limit molecular weight, was determined. Next, the following correlation equation of a diameter and a molecular weight of a spherical protein in a neutral buffer was substituted with the exclusion limit molecular weight, and the calculated value was defined as the maximum diameter of the pores of sample particles.

Diameter of spherical protein in neutral buffer solution (angstrom) = 2.523 × (molecular weight)^{0.3267}

(6) Average pore diameter

The above-described correlation equation of a diameter and a molecular weight of a spherical protein in a neutral buffer solution was substituted with the molecular weight which corresponded to a value of [the maximum $K_{av}$] /2 in the portion showing linearity, and the calculated value was defined as the average diameter of the pores of sample particles.

Additionally, when a value of $K_{av}$ of the adsorbent for a target substance to be adsorbed is measured, an accurate measurement may possibly become impossible due to an adsorption of the target substance to be adsorbed on the adsorbent. Accordingly, a value of $K_{av}$ of the adsorbent for a target substance to be adsorbed was determined by measuring values of $K_{av}$ of two or more kinds of proteins having molecular weights close to that of a target substance to be adsorbed and carrying out calculation from the obtained data. For example, when a target substance to be adsorbed was IgG, a value of $K_{av}$ was determined from data of ferritin and albumin.

Test Example 3: Measurement of average particle diameter

A median diameter was measured by using a laser diffraction/scattering particle size distribution analyzer (LA-950, manufactured by Horiba Ltd.). The measured value was defined as an average particle diameter.

Test Example 4: Measurement of static binding capacity

A static binding capacity was determined by the following procedures:

a solution was obtained by dissolving 70 mg of human polyclonal IgG (0.467 mL of 1500 mg / 10 mL Gamma-Globulin NICHIYAKU, produced by Nichiyaku) in a phosphate buffer (produced by Sigma) having a pH of 7.4 and adjusting the entire amount to be 35 mL;

the above solution was brought into contact with 0.5 mL of the adsorbent of which solvent was substituted with a phosphate buffer (produced by Sigma) having a pH of 7.4;

the mixture was stirred at 25°C for 2 hours; and

the decreased amount of IgG in the supernatant was measured.

Test Example 5: Measurement of dynamic binding capacity

(1) Preparation of solution

The following solutions were prepared.
A solution: a phosphate buffer having a pH of 7.4 (produced by Sigma)
B solution: 35 mM sodium acetate having a pH of 3.5 (prepared from acetic acid and sodium acetate produced by Nacalai Tesque, Inc. and RO water)
C solution: 1 M acetic acid (prepared from acetic acid produced by Nacalai Tesque, Inc. and RO water)
D solution: 1 mg/mL human polyclonal IgG solution (prepared from 1500 mg / 10 mL Gamma-Globulin NICHIYAKU, produced by Nichiyaku and the A solution)
E solution: 6 M urea (prepared from urea produced by KANTO CHEMICAL CO., INC. and RO water)

The respective solutions were defoamed before use.

(2) Packing and preparation

As a column chromatography apparatus, AKTA explorer 100 (manufactured by GE Healthcare Bioscience) was used. A mesh of 22 $\mu$m was attached to a column having a diameter of 0.5 cm and a height of 15 cm. Into the column, 3 mL of each adsorbent of the present invention was loaded and packed by passing a 20% ethanol aqueous solution (prepared from ethanol produced by Wako Pure Chemical Industries, Ltd. and RO water) at a linear rate of 450 cm/hr for 1 hour. Sampling tubes having a volume of 15 mL were set to a fraction collector, and sampling tubes for an effluent were previously filled with the neutralizing solution.

(3) Purification of IgG

The A solution in an amount of 9 mL was allowed to pass at a linear rate of 300 cm/hr and subsequently, while monitoring UV, the D solution was allowed to pass at a linear rate of 300 cm/hr until 10% of IgG was eluted. Next, 30 mL of the A solution was allowed to pass at a linear rate of 300 cm/hr and 30 mL of the B solution was allowed to pass at a linear rate of 300 cm/hr to elute IgG. Subsequently, 9 mL of the C solution was allowed to pass at linear rate of 300 cm/hr and 9 mL of the E solution was allowed to pass at linear rate of 300 cm/hr.

Test Example 6: Measurement of compressive stress

A slurry containing 50 vol% of the porous particle or the adsorbent was loaded to a graduated cylinder which was made of glass and which had an inner diameter of 15 mm. The porous particle or adsorbent was deposited to be packed until the volume of the porous particle or adsorbent was not further decreased by applying vibration to the graduated cylinder made of glass, and the amount of the porous particle or adsorbent was adjusted as to set the volume of the porous particle or adsorbent to be 4 mL. The volume at the time was defined as the initial volume. A piston made of a metal (which did not cause friction with the inner wall of the graduated cylinder and was processed to prevent elution of the porous particle or adsorbent) was attached to an autograph (EZ-TEST, manufactured by SHIMADZU CORPORATION) equipped with a load cell for 20N. The position of the piston was adjusted so that the bottom surface of the piston was moved to the position corresponding to 120 vol% of the porous particle or adsorbent. The piston was lowered at a testing rate of 5 mm/min in a manner of preventing entrainment of bubbles, and the porous particle or adsorbent was compressed and the volume was decreased. The compressive stress at arbitrary positions was measured.

Comparative Example 1

Crystalline cellulose (2.73 g) was dissolved in 1-ethyl-3-methylimidazolium acetate (produced by Sigma Aldrich, 20 g) at 70°C to prepare a 12.0 wt% solution. From the cellulose solution, an amount of 15 mL was measured to be taken out. The measured amount of the solution was added to medium chain fatty acid triglyceride (MCT) (Acta M-2, fatty acid composition: 100% of C8, 85 mL, produced by Riken Vitamin Co., Ltd.) that was stirred at a rate of 4000 rpm by a homogenizer (HG-200, manufactured by HSIANGTAI). The cellulose solution was dispersed in MCT by stirring for 5 minutes. The dispersion was gradually added to water at 25°C that was stirred at a rate of 300 rpm to solidify the cellulose. The dispersion was settled to separate a liquid phase. As a result, a bilayer solution composed of an upper layer containing MCT and a lower layer containing water and 1-ethyl-3-methylimidazolium acetate was observed. The cellulose was deposited in the lower layer.

The cellulose was recovered and washed with methanol and thereafter washed with water. It was found by observation with a microscope (Digital Microscope VH-6200, manufactured by Keyence Corporation) that particulate cellulose was obtained.

The microscopic image of the obtained particles was analyzed by using a commercially available image analyzing processing software (Motic Images Plus, manufactured by Motic) to measure an average aspect ratio. The average aspect ratio was determined by calculating the aspect ratio of each particle from the measured major axis and minor axis of the particles in the image and calculating the average of the calculated ratios. A median diameter was measured by using a laser diffraction/scattering particle size distribution analyzer (LA-950, manufactured by Horiba Ltd.) and employed the measured value as an average particle diameter. As a result, it was proved that fine cellulose particles with complete sphericity and having an average particle diameter of 104.0 $\mu$m and an average aspect ratio of 1.10 were obtained.

The hardness of the cellulose particle was evaluated by measuring the compressive strength with the use of a compressive strength testing apparatus (EZ-TEST, manufactured by SHIMADZU CORPORATION). As a result, when the volume was compressed by 10%, the stress was 0.042 Mpa and it was apparent that the cellulose particle had proper hardness.

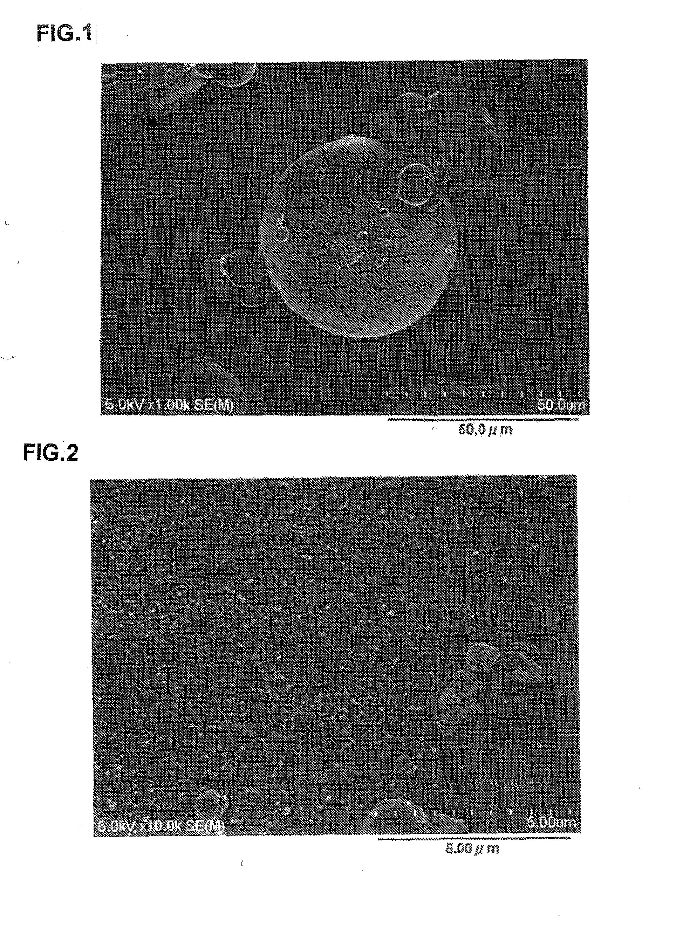

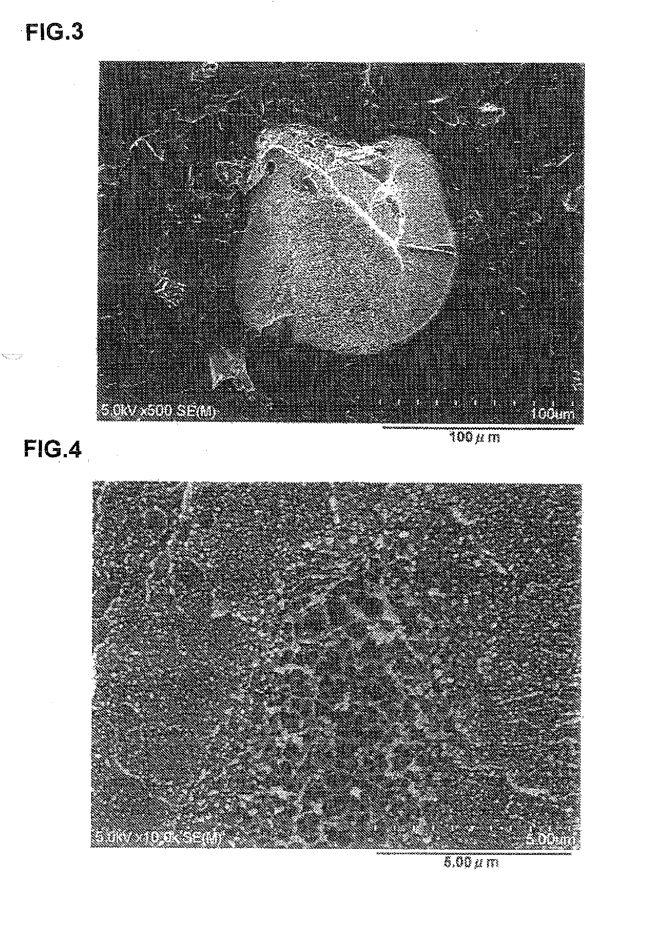

Further, the inside of the pores of the obtained cellulose particles was replaced with 2-methyl-2-propanol, and the obtained cellulose particles were dried by freeze drying and then analyzed with a scanning electron microscope (S-800, manufactured by Hitachi Ltd., hereinafter referred to as "SEM") . As a result, the cellulose particles were confirmed to be porous particles as shown in Figs. 1 to 4.

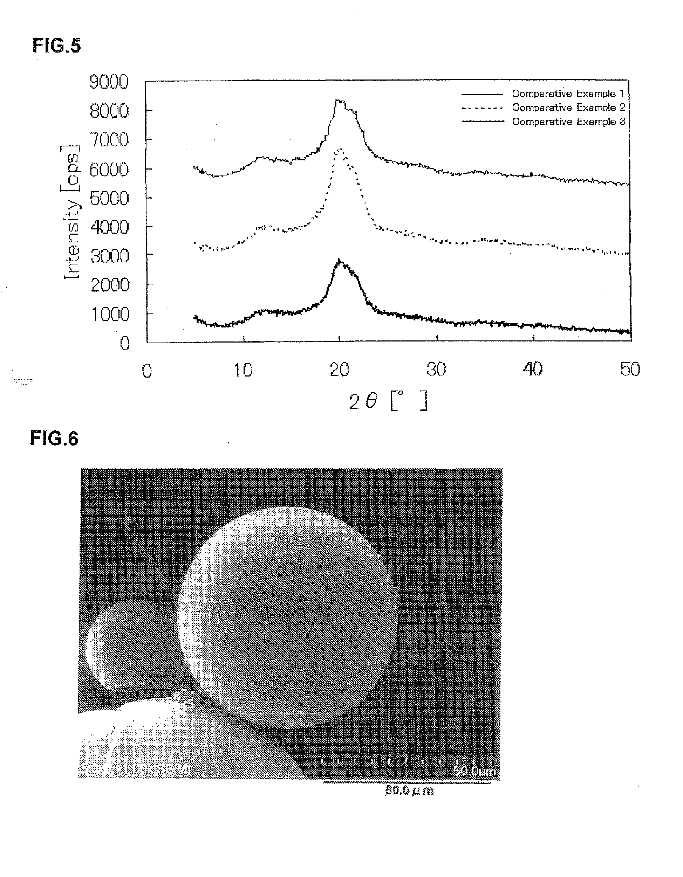

In addition, the dried cellulose particles were analyzed by powder x-ray diffraction (XRD, Rigaku Mini Flex II, the same applies hereinafter). The results are shown in Fig. 5. As shown in Fig. 5, it was confirmed that the cellulose particles were crystalline.

Comparative Example 2

Cellulose particles were precipitated in the same condition and same manner as in the Comparative Example 1, except that the cellulose concentration was changed to 10.0 wt%. As a result, cellulose particles having an average particle diameter of 121.4 $\mu$m and an average aspect ratio of 1.12 were obtained. When the cellulose volume was compressed by 10%, the stress was 0.015 Mpa. The obtained particles were porous particles similar to those of the Comparative Example 1.

Comparative Example 3

Cellulose particles were precipitated in the same condition and same manner as in the Comparative Example 1, except that the cellulose concentration was changed to 7.0 wt%. As a result, cellulose particles having an average particle diameter of 104.0 $\mu$m and an average aspect ratio of 1.10 were obtained. When the cellulose volume was compressed by 10%, the stress was 0.004 Mpa. The obtained particles were porous particles similar to those of the Comparative Example 1. Fig. 5 shows the result of analysis of the cellulose particles of the Comparative Examples 1 to 3 by XRD, and it could be confirmed that all particles were crystalline and thus, it could be judged that the cellulose particles obtained from water had crystallinity.

Example 1

Cellulose particles were precipitated in the same condition and same manner as in the Comparative Example 1, except that the cellulose concentration was changed to 12.0 wt% and the cellulose-insoluble liquid was changed to a mixed solvent of methanol and water at a ratio of methanol : water = 7 : 3. After the cellulose precipitation, the mixture was settled to give a bilayer solution composed of a lower layer containing MCT and an upper layer containing methanol, water, 1-ethyl-3-methylimidazolium acetate and MCT. The precipitated cellulose was deposited in the upper layer. The cellulose was recovered, and it was found that the obtained cellulose was spherical cellulose particle having an average particle diameter of 98.5 $\mu$m and an average aspect ratio of 1.07. As shown in SEM photographs of Figs. 6 to 9, the particles were porous.

Example 2

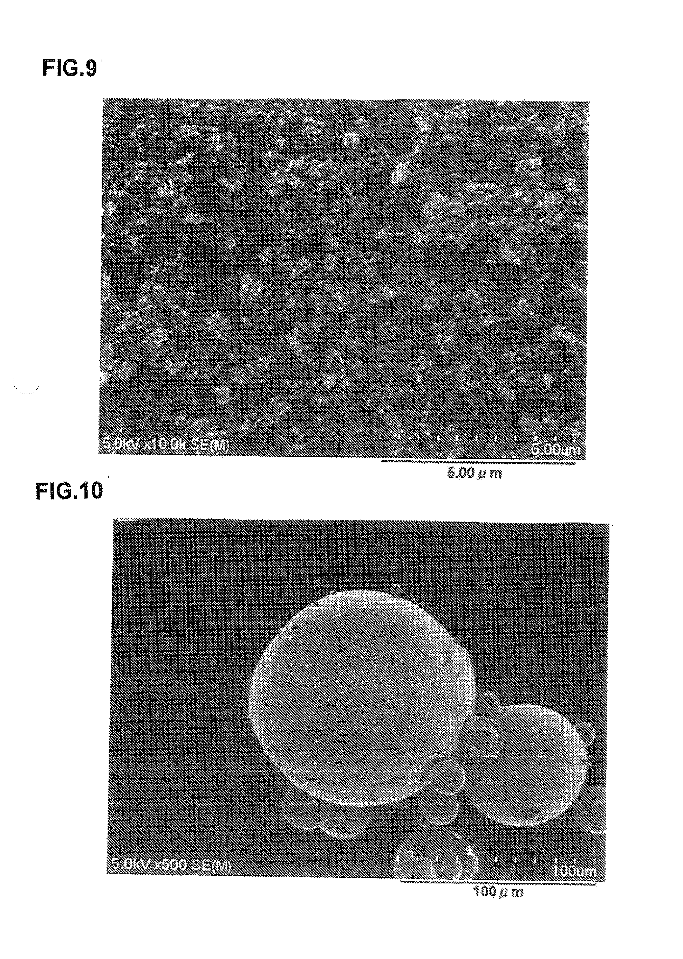

Cellulose particles were precipitated in the same condition and same manner as in the Comparative Example 1, except that the cellulose concentration was changed to 12.0 wt% and the cellulose-insoluble liquid was changed to a mixed solvent of methanol and water at a ratio of methanol : water = 9 : 1. After the cellulose precipitation, the mixture was settled to give a bilayer solution composed of a lower layer containing MCT and an upper layer containing methanol, water, 1-ethyl-3-methylimidazolium acetate and MCT. The precipitated cellulose was deposited in the upper layer. The cellulose was recovered, and it was found that the obtained cellulose was spherical cellulose particle having an average particle diameter of 97.3 $\mu$m and an average aspect ratio of 1.07. As shown in SEM photographs of Figs. 10 to 13, the particles were porous.

Example 3

Cellulose particles were precipitated in the same condition and same manner as in the Comparative Example 1, except that the cellulose-insoluble liquid was changed to methanol. After a cellulose droplet dispersion was added to methanol in order to precipitate cellulose, the solution was settled to give a bilayer solution composed of a lower layer containing MCT and an upper layer containing methanol, 1-ethyl-3-methylimidazolium acetate and MCT. The precipitated cellulose was deposited in the upper layer. The cellulose was recovered, and it was found that the obtained cellulose was spherical cellulose particle having an average particle diameter of 60.4 $\mu$m and an average aspect ratio of 1.07.

When the cellulose volume was compressed by 10%, the stress was 0.053 Mpa.

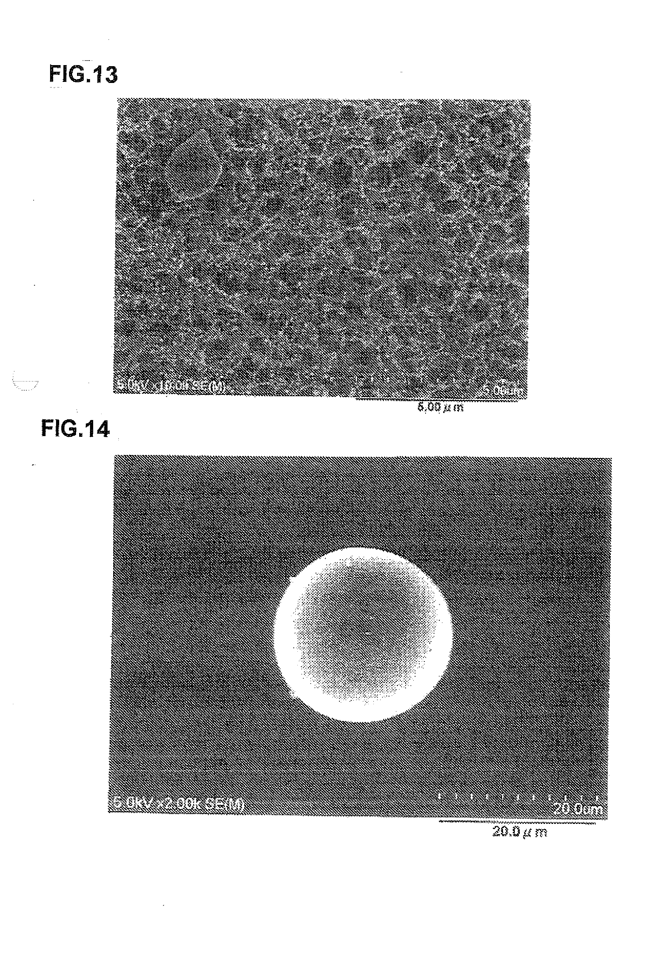

As shown in SEM photographs of Figs. 14 to 17, the particles were porous.

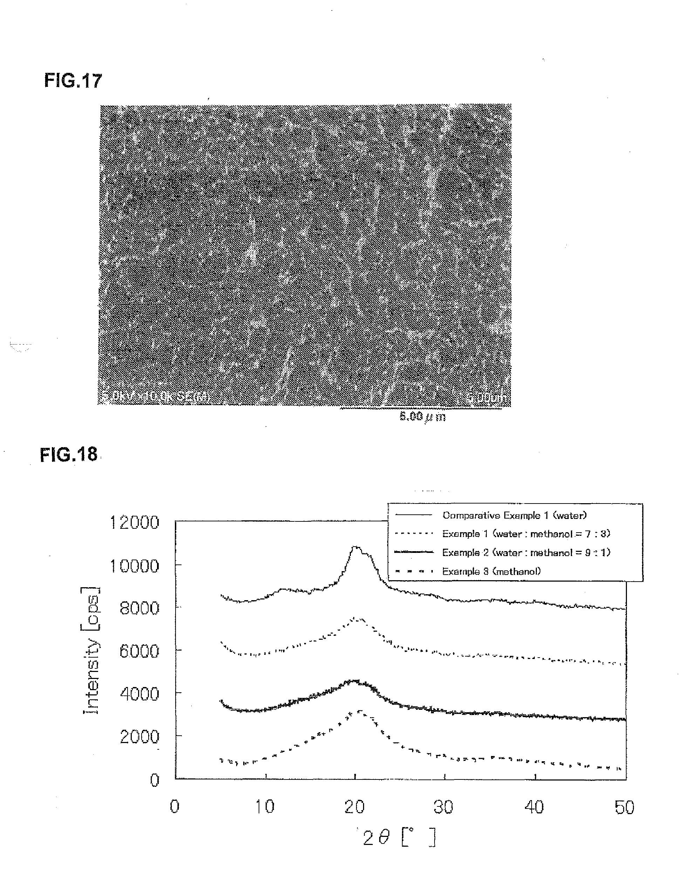

After freeze drying, XRD analysis was carried out to find that the obtained cellulose particles were amorphous. Fig. 18 shows the results of XRD analysis for the cellulose particles obtained in the Comparative Example 1 and Examples 1 to 3, and it could be judged that the cellulose particles were amorphous, except those obtained by using water as a solidification liquid in the Comparative Example 1.

When the used solvents were recovered and methanol was evaporated using an evaporator, 1-ethyl-3-methylimidazolium acetate and MCT were separated. After MCT was removed, the volume and weight of 1-ethyl-3-methylimidazolium acetate were measured to find that the concentration of 1-ethyl-3-methylimidazolium acetate was 98%. The ionic liquid with high purity obtained as described above can sufficiently be reused.

Example 4

Cellulose particles were precipitated in the same condition and same manner as in the Example 3, except that the cellulose concentration was changed to 10.0 wt%. As a result, obtained were spherical cellulose particles having an average particle diameter of 54.23 $\mu$m and an average aspect ratio of 1.07. When the cellulose volume was compressed by 10%, the stress was 0.027 Mpa.

Example 5

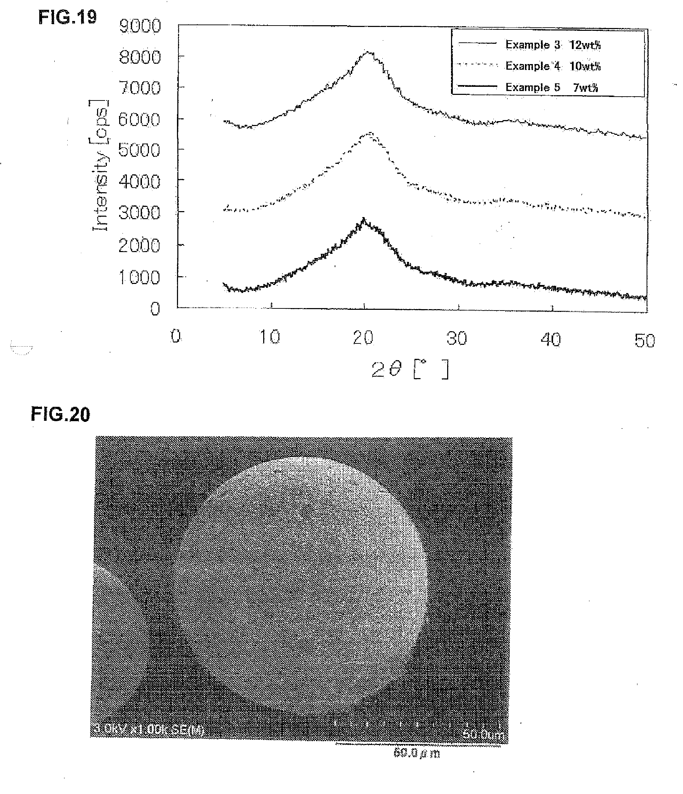

Cellulose particles were precipitated in the same condition and same manner as in the Example 3, except that the cellulose concentration was changed to 7.0 wt%. As a result, obtained were spherical cellulose particles having an average particle diameter of 60.36 $\mu$m and an average aspect ratio of 1.07. Fig. 19 shows the results of XRD analysis for the cellulose particles obtained in the Examples 3 to 5, and all of the cellulose particles were amorphous.

Example 6

Cellulose particles were precipitated in the same condition and same manner as in the Comparative Example 1, except that the cellulose concentration was changed to 12.0 wt% and the cellulose-insoluble liquid was changed to ethanol. As a result, obtained were spherical cellulose particles having an average particle diameter of 87.44 $\mu$m and an average aspect ratio of 1.07. Different from the case of using water or methanol, in the case of using ethanol, the solution was not separated into two layers.

When the cellulose volume was compressed by 10%, the stress was 0.026 Mpa.

After freeze drying, XRD analysis was carried out to find that the obtained cellulose particles were amorphous. From Figs. 5 and 19, it was found that the crystallinity of the cellulose particles was independent of the cellulose solution concentration and that the cellulose particles became amorphous in the case of using an alcohol as the cellulose-insoluble liquid and became crystalline in the case of using water. The crystallinity of the cellulose particles could be controlled depending on the kind of the cellulose-insoluble liquid.

Example 7

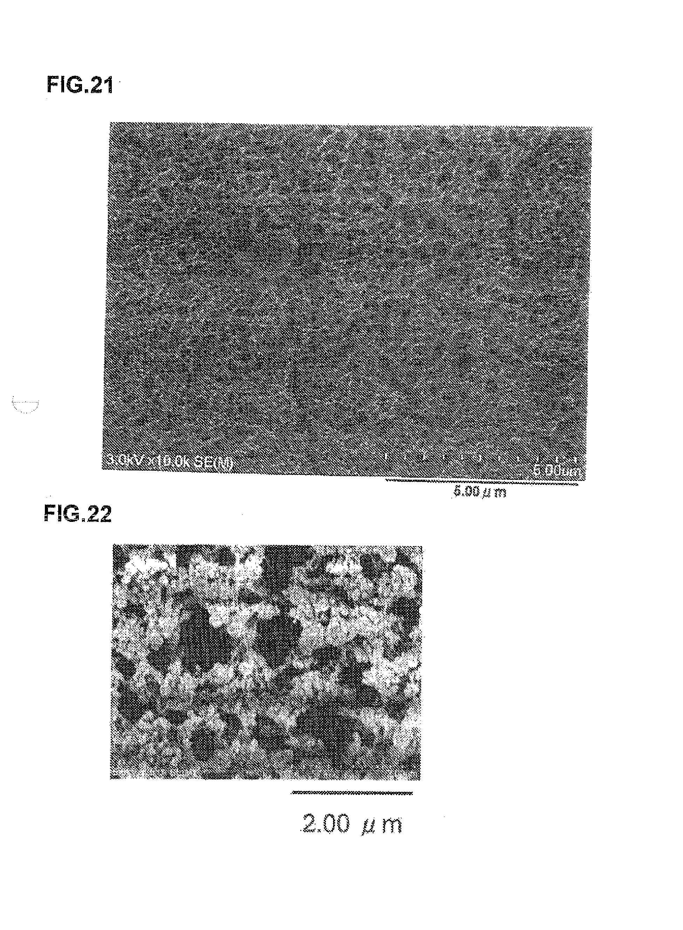

Crystalline cellulose (5.80 g) was dissolved in 1-ethyl-3-methylimidazolium acetate (52.18 g) at 70°C to prepare a 10.0 wt% solution. From the cellulose solution, a volume of 50 mL was measured to be taken out. The measured amount of the solution was added to 950 mL of MCT that was stirred at a rate of 100 rpm by an H type blade having a blade diameter of 70 mm in a cylindrical container having an inner diameter of 113 mm, in order to be dispersed. The temperature of the dispersion was adjusted to be 40°C, and the dispersion was allowed to pass at a rate of 1 L/minute through a static mixer (Bunsankun 1.5A, manufactured by Fujikin). Then, the dispersion was added to a mixed solvent of methanol and water at a ratio of methanol : water = 9 : 1, which was a cellulose-insoluble liquid, to precipitate the cellulose. The mixed solvent of methanol : water = 9 : 1 was stirred at a rate of 300 rpm by a flat turbine blade having a blade diameter of 64.2 mm in a cylindrical container having an inner diameter of 143.5 mm, and the solution volume was adjusted to be 2000 mL and the solution temperature was adjusted to be 25°C. The cellulose was recovered and washed with methanol and thereafter washed with water. Similarly to the Comparative Example 1, the obtained cellulose was observed with a microscope to find that obtained were cellulose particles having an average particle diameter of 86.3 $\mu$m and an average aspect ratio of 1.07. As shown in SEM photographs of Figs. 20 and 21, the particles were porous.

Example 8

Crystalline cellulose in an amount of 2.73 g was dissolved in a solution obtained by adding 2.22 g of a mixed solution of methanol and water at a ratio of methanol : water = 7 : 3 to 20 g of 1-ethyl-3-methylimidazolium acetate (produced by Sigma Aldrich). The cellulose concentration in the solution was 10.9 wt%. From the cellulose solution, a volume of 15 mL was measured to be taken out. The measured amount of the solution was added to 85 mL of medium chain fatty acid triglyceride (MCT) (trade name: Acta M-2, fatty acid composition: 100% of C8, produced by Riken Vitamin Co., Ltd.) that was stirred at a rate of 400 rpm at 50°C. After the cellulose solution was dispersed in MCT by stirring for 30 minutes, the cellulose droplet dispersion was added to 400 mL of a mixed solvent of methanol and water at a ratio of methanol : water = 7 : 3 that was stirred at 300 rpm at 50°C to solidify cellulose.

After the mixture was stirred for 30 minutes, cellulose particles were recovered and washed with ethanol and thereafter washed with water. Next, the cellulose particles were classified using a mesh of 125 $\mu$m to remove particles having a large particle diameter, and then classified using a mesh of 53 $\mu$m to remove particles having a small particle diameter. Further, a portion of the obtained cellulose particles was taken out, and the solvent thereof was substituted with 2-methyl-2-propanol. The obtained cellulose particles were dried by freeze drying, and then analyzed with a scanning electron microscope (S-4800, manufactured by Hitachi Ltd. , hereinafter referred to as "SEM"). As a result, the cellulose particles were confirmed to be porous particles as shown in Fig. 22.

The fine pore diameter of the obtained cellulose porous particles was determined by the above-described $K_{av}$ measurement method; as a result, the maximum fine pore diameter and average diameter of the cellulose porous particles were found to be 725 angstroms and 271 angstroms, respectively. The inclination k was -0.153. A value of $K_{av}$ measured by using IgG was 0.86. The exclusion limit molecular weight was $3.5 \times 10^7$. Further, the dried cellulose particles were analyzed by powder x-ray diffraction (XRD, Rigaku Mini Flex II, the same applies hereinafter), and the result showed an amorphous pattern. The average particle diameter was 94 $\mu$m.

Example 9

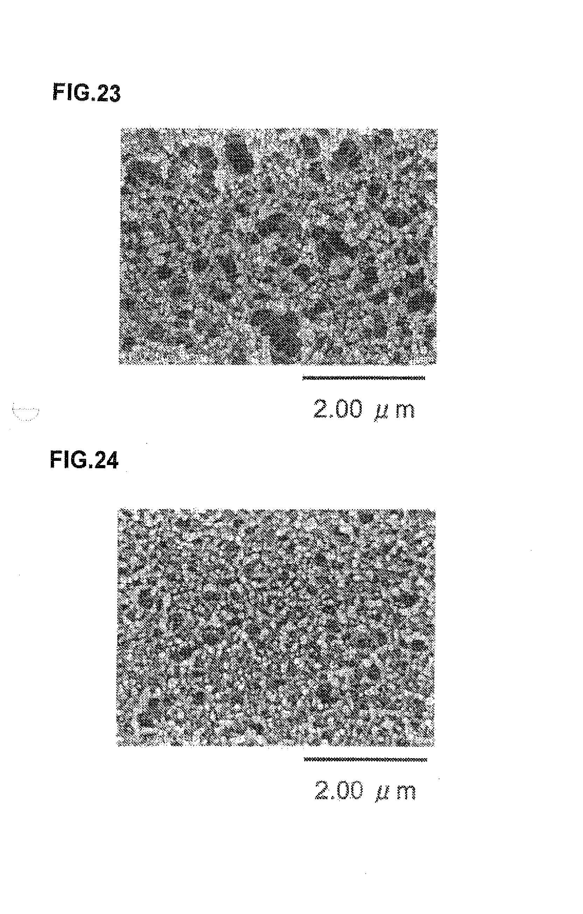

Cellulose particles were obtained in the same condition as in the Example 8, except that the temperature of MCT was changed to 25°C and the temperature of the mixed solvent of methanol : water = 7 : 3 was changed to 25°C. As the result of analysis by SEM, the obtained cellulose particles were confirmed to be porous particles as shown in Fig. 23. The fine pore diameter of the obtained cellulose porous particles was determined by the above-described $K_{av}$ measurement method; as a result, the maximum fine pore diameter and average diameter of the cellulose porous particles were found to be 350 angstroms and 194 angstroms, respectively. The inclination k was -0.266. The value of $K_{av}$ measured by using IgG was 0.93. The exclusion limit molecular weight was $3.8 \times 10^6$. The average particle diameter was 88 $\mu$m.

Example 10

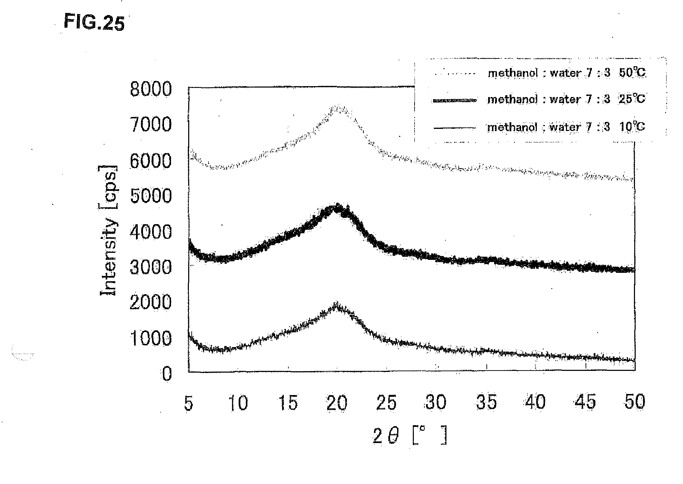

Cellulose particles were obtained in the same condition as in the Example 8, except that the temperature of MCT was changed to 10°C and the temperature of the mixed solvent of methanol : water = 7 : 3 was changed to 10°C. As the result of analysis by SEM, the obtained cellulose particles were confirmed to be porous particles as shown in Fig. 24. The average particle diameter was 83 $\mu$m. Fig. 25 shows the results of XRD analysis for the cellulose particles obtained in the Examples 8 to 10, and all of the cellulose particles were amorphous.

Example 11

Cellulose particles were obtained in the same condition as in the Example 9, except that 2.22 g of a mixed solution of methanol and water at a ratio of methanol : water = 6 : 4 was added to 20 g of 1-ethyl-3-methylimidazolium acetate (produced by Sigma Aldrich) and that the cellulose droplet dispersion was added to 400 mL of a cellulose-insoluble liquid which was a mixed solvent of methanol and water at a ratio of methanol : water = 6 : 4. As the result of analysis by SEM, the obtained cellulose particles were confirmed to be porous particles similar to the particles of the Example 9. The fine pore diameter of the obtained cellulose porous particles was determined by the above-described $K_{av}$ measurement method; as a result, the maximum fine pore diameter and average diameter of the cellulose porous particles were found to be 362 angstroms and 201 angstroms, respectively. The inclination k was -0.222. The value of $K_{av}$ measured by using IgG was 0.80. The exclusion limit molecular weight was $4.2 \times 10^6$. XRD analysis was carried out to find that the obtained cellulose particles were amorphous. The average particle diameter was 90 $\mu$m.

Example 12

Cellulose particles were obtained in the same condition as in the Example 9, except that 2.22 g of a mixed solvent of methanol and water at a ratio of methanol : water = 9 : 1 was added to 20 g of 1-ethyl-3-methylimidazolium acetate (produced by Sigma Aldrich) and that the cellulose droplet dispersion was added to 400 mL of a cellulose-insoluble liquid which was a mixed solvent of methanol and water at a ratio of methanol : water = 9 : 1. As the result of analysis by SEM, the obtained cellulose particles were confirmed to be porous particles similar to the particles of the Example 9. The fine pore diameter of the obtained cellulose porous particles was determined by the above-described $K_{av}$ measurement method; as a result, the maximum fine pore diameter and average diameter of the cellulose porous particles were found to be 261 angstroms and 184 angstroms, respectively. The inclination k was -0.380. The value of $K_{av}$ measured by using IgG was 0.86. The exclusion limit molecular weight was $1.5 \times 10^6$. XRD analysis was carried out to find that the obtained cellulose particles were amorphous. The average particle diameter was 90 $\mu$m.

Example 13

Cellulose particles were obtained in the same condition as in the Example 9, except that 1.93 g of crystalline cellulose was dissolved in a solution obtained by adding 2.22 g of a mixed solvent of methanol and water at a ratio of methanol : water = 7 : 3 to 20 g of 1-ethyl-3-methylimidazolium acetate (produced by Sigma Aldrich) and that the cellulose concentration was changed to 8.0 wt%. As the result of analysis by SEM, the obtained cellulose particles were confirmed to be porous particles similar to the particles of the Example 8. The fine pore diameter of the obtained cellulose porous particles was determined by the above-described $K_{av}$ measurement method; as a result, the maximum fine pore diameter and average diameter of the cellulose porous particles were found to be 1020 angstroms and 306 angstroms, respectively. The inclination k was -0.117. The value of $K_{av}$ measured by using IgG was 0.79. The exclusion limit molecular weight was $1.0 \times 10^8$. XRD analysis was carried out to find that the obtained cellulose particles were amorphous. The average particle diameter was 94 $\mu$m.

Example 14

Crystalline cellulose in an amount of 2.00 g was dissolved in 18.0 g of 1-ethyl-3-methylimidazolium acetate to obtain a solution having a cellulose concentration of 10.0 wt%. From the cellulose solution, a volume of 15 mL was measured to be taken out. The measured amount of the solution was added to 85 mL of MCT that was stirred at a rate of 400 rpm at 25°C. After the cellulose solution was dispersed in MCT by stirring for 30 minutes, the cellulose droplet dispersion was added to 400 mL of a mixed solvent of methanol and water at a ratio of methanol : water = 7 : 3 that was stirred at 300 rpm at 25°C to solidify cellulose.
After the mixture was stirred for 30 minutes, cellulose particles were recovered and washed with ethanol and thereafter washed with water.
The fine pore diameter of the obtained cellulose porous particles was determined by the above-described $K_{av}$ measurement method; as a result, the maximum fine pore diameter and average diameter of the cellulose porous particles were found to be 1340 angstroms and 353 angstroms, respectively. The inclination k was -0.102. The value of $K_{av}$ measured by using IgG was 0.78. The exclusion limit molecular weight was $2.3 \times 10^8$.

Example 15: Production of crosslinked porous particle

From the cellulose porous particles obtained in the Example 8, coarse porous particles were removed by using a sieve having an opening of 125 $\mu$m, and then too small porous particles were removed by using a sieve having an opening of 53 $\mu$m. RO water was added to 11.0 mL of the cellulose porous particles A after the classification operation to adjust the volume to be 16.5 mL, and the mixture was transferred to a 50 mL centrifugal tube. Further, 3.86 mL of 4N NaOH (prepared from NaOH produced by Nacalai Tesque, Inc. and RO water) was added thereto and the obtained mixture was heated to 40°C in a hybridization oven (MHS-2000, manufactured by TOKYO RIKAKIKAI CO., LTD.). To the mixture, 1.77 g of Denacol EX-314 containing glycerol polyglycidyl ether (produced by Nagase ChemteX Corporation) was added as a crosslinking agent. The mixture was stirred at 40°C for 4 hours. After the completion of the reaction, while carrying out filtration under reduced pressure on a glass filter (11GP 100, manufactured by SIBATA SCIENTIFIC TECHNOLOGY LTD.), the obtained porous particles were washed with RO water in a volume of 20 times as much as that of the obtained porous particles, to obtain crosslinked porous particles.
RO water was added to the obtained crosslinked porous particles to increase the total volume to 10 times as much as that of the crosslinked porous particles, and the mixture was sealed with two sheets of an aluminum foil and heated at 120°C for 1 hour by using an autoclave (High pressure sterilizer ES-315, manufactured by TOMY SEIKO CO., LTD.). The mixture was allowed to cool down to room temperature, and then the porous particles were washed with RO water in a volume of 5 times as much as that of the porous particles on a glass filter (11GP 100, manufactured by SIBATA SCIENTIFIC TECHNOLOGY LTD.), to obtain autoclaved porous particles B of which epoxy groups were transformed into glyceryl groups.
Next, RO water was added to the autoclaved porous particles B (10 mL) to adjust the volume to be 16.5 mL, and the mixture was transferred to a 50 mL centrifugal tube. Further, 3.86 mL of 4N NaOH (prepared from NaOH produced by Nacalai Tesque, Inc. and RO water) was added thereto, and the obtained mixture was heated to 40°C in a hybridization oven (MHS-2000, manufactured by TOKYO RIKAKIKAI CO., LTD.). Then, 1.77 g of Denacol EX-314 (produced by Nagase ChemteX Corporation) was added thereto, and the mixture was stirred at 40°C for 4 hours. After the completion of the reaction, while carrying out filtration under reduced pressure on a glass filter (11GP 100, manufactured by SIBATA SCIENTIFIC TECHNOLOGY LTD.), the obtained porous particles were washed with RO water in a volume of 20 times as much as that of the obtained porous particles, to obtain crosslinked porous particles.

RO water was added to the obtained crosslinked porous particles to increase the total volume to 10 times as much as that of the crosslinked porous particles, and the mixture was put into an Erlenmeyer flask (300 mL) made of glass. The flask was sealed with two sheets of an aluminum foil, and the mixture was heated at 120°C for 60 minutes by using an autoclave (High pressure sterilizer ES-315, manufactured by TOMY SEIKO CO., LTD.). The mixture was allowed to cool down to room temperature, and then the porous particles were washed with RO water in a volume of 5 times as much as that of the porous particles on a glass filter (11GP 100, manufactured by SIBATA SCIENTIFIC TECHNOLOGY LTD.), to obtain autoclaved porous particles C.

Further, RO water was added to the autoclaved porous particles C (10.5 mL) to adjust the volume to be 15.8 mL, and the mixture was transferred to a 50 mL centrifugal tube. Further, 2.68 mL of 4N NaOH (prepared from NaOH produced by Nacalai Tesque, Inc. and RO water) was added thereto, and the obtained mixture was heated to 40°C in a hybridization oven (MHS-2000, manufactured by TOKYO RIKAKIKAI CO., LTD.). Then, 1.69 g of Denacol EX-314 (produced by Nagase ChemteX Corporation) was added thereto, and the mixture was stirred at 40°C for 4 hours. After the completion of the reaction, while carrying out filtration under reduced pressure on a glass filter (11GP100, manufactured by SIBATASCIENTIFIC TECHNOLOGY LTD.), the obtained porous particles were washed with RO water in a volume of 20 times as much as that of the obtained porous particles, to obtain crosslinked porous particles.

RO water was added to the obtained crosslinked porous particles to increase the total volume to 10 times as much as that of the crosslinked porous particles, and the mixture was put into an Erlenmeyer flask (300 mL) made of glass. The flask was sealed with two sheets of an aluminum foil, and the mixture was heated at 120°C for 60 minutes by using an autoclave (High pressure sterilizer ES-315, manufactured by TOMY SEIKO CO., LTD.). The mixture was allowed to cool down to room temperature, and then the porous particles were washed with RO water in a volume of 5 times as much as that of the porous particles on a glass filter (11GP 100, manufactured by SIBATA SCIENTIFIC TECHNOLOGY LTD.), to obtain autoclaved porous particles D.

Example 16: Production of adsorbent

RO water was added to 11.0 mL of the crosslinked porous particles D obtained in the Example 15 to adjust the total volume to be 17.0 mL. The mixture was transferred to a 50 mL centrifugal tube. The centrifugal tube was installed in a mix rotor (Mix Rotor MR-3, manufactured by AS ONE Corporation) and the mixture was stirred at 25°C. Next, 6.0 mL of a 8.64 mg/mL sodium periodate aqueous solution was prepared by dissolving sodium periodate (produced by Wako Pure Chemical Industries, Ltd.) in RO water and added to the centrifugal tube containing the porous particles D. The mixture was stirred at 25°C for 1 hour. After the reaction, the porous particles were washed with RO water on a glass filter (11GP 100, manufactured by SIBATA SCIENTIFIC TECHNOLOGY LTD.) until the electric conductivity of the filtrate became not more than 1 $\mu$S/cm, to obtain a formyl group-crosslinked porous carrier E. The electric conductivity of the washing filtrate was measured by a conductivity meter (ECTester 10 Pure+, manufactured by EUTECH INSTRUMENTS). A formyl group content in the obtained porous particles E was measured by the above-described method to find that the formyl group content was 20.17 $\mu$mol per 1 mL of the porous particles E.

The solvent of the obtained formyl-crosslinked porous carrier E (9.0 mL) was substituted with 30 mL of a buffer of which pH was 11 and which contained 0.5 M trisodium citrate (produced by KANTO CHEMICAL CO., INC.) and 0.15 M sodium chloride (produced by KANTO CHEMICAL CO., INC.) on a glass filter (11GP 100, manufactured by SIBATA SCIENTIFIC TECHNOLOGY LTD.). The formyl group-containing porous carrier after substitution was put into a centrifugal tube, and the amount was adjusted to be 14.0 mL in total by volume. After 5.327 g of a protein A-containing solution (PNXL 30, produced by KANEKA CORPORATION) which was produced by the method disclosed in WO 2006/004067 and of which concentration of protein A was 67.58 mg/mL was added thereto, the pH was adjusted to be 12 by using 0.08N NaOH (prepared from one produced by Nacalai Tesque, Inc. and RO water) at 6°C. Then, the mixture was stirred at 6°C for 23 hours by using a mix rotor (Mix Rotor MR-3, manufactured by AS ONE Corporation) to progress a reaction.

After the reaction for 23 hours, the pH of the reaction mixture was adjusted to be 5.0 by using 2.4 N citric acid (prepared from citric acid produced by KANTO CHEMICAL CO., INC. and RO water), and then the mixture was stirred at 6°C for 4 hours by using a mix rotor (Mix Rotor MR-3, manufactured by AS ONE Corporation). Continuously, 0.39 mL of a 5.5% dimethylamine borane (DMAB) aqueous solution (prepared from dimethylamine borane produced by Kishida Chemical Co. , Ltd. and RO water) was added thereto, and the mixture was stirred at 6°C for 1 hour. Then, the reaction temperature was increased to 25°C and the mixture was stirred at 25°C for 18 hours using a mix rotor (Mix Rotor MR-3, manufactured by AS ONE Corporation) to carry out a reaction. After the completion of the reaction, the maximum UV absorbance of the reaction mixture around 278 nm was measured to find that the amount of the protein A as an affinity ligand to be introduced was 28.93 mg per 1 mL of the porous carrier.

The porous carrier after the reaction was washed with RO water in a volume of 3 times as much as that of the porous carrier on a glass filter (11GP 100, manufactured by SIBATA SCIENTIFIC TECHNOLOGY LTD.). Next, 0.1 N citric acid monohydrate (prepared from citric acid monohydrate produced by KANTO CHEMICAL CO., INC. and RO water) in a 3 times amount by volume was added, and further 0.1 N citric acid monohydrate was added to the porous carrier to adjust the total volume to be not less than 30 mL. The mixture was put into a centrifugal tube and washed with an acid while stirred at 25°C for 30 minutes.

After the above acid washing, the porous carrier was washed with RO water in a volume of 3 times as much as that of the porous carrier on a glass filter (11GP 100, manufactured by SIBATA SCIENTIFIC TECHNOLOGY LTD.), and thereafter an aqueous solution of 0.05 M sodium hydroxide and 1 M sodium sulfate (prepared from sodium hydroxide produced by Nacalai Tesque, Inc., sodium sulfate produced by KANTO CHEMICAL CO., INC., and RO water) was added in a 3 times amount by volume. Next, an aqueous solution of 0.05 M sodium hydroxide and 1 M sodium sulfate was added to the porous carrier so as that the total volume was adjusted to be not lower than 30 mL. The mixture was put into a centrifugal tube and washed with alkali while stirred at room temperature for 30 minutes.

After the above alkali washing, the porous carrier was washed with RO water in a volume of 20 times as much as that of the porous carrier on a glass filter (11GP 100, manufactured by SIBATA SCIENTIFIC TECHNOLOGY LTD.). Next, 0.1 N citric acid monohydrate (prepared from citric acid monohydrate produced by KANTO CHEMICAL CO., INC. and RO water) in a volume of 3 time as much as that of the porous carrier was added, and it was confirmed that the filtrate became neutral. Then, the porous carrier was washed by using RO water until the electric conductivity of the washing filtrate became not more than 1 μS/cm, to obtain an adsorbent F as a target adsorbent on which a protein A was immobilized. The electric conductivity of the washing filtrate was measured by a conductivity meter (ECTester 10 Pure+, manufactured by EUTECH INSTRUMENTS).

As a target substance to be adsorbed by the obtained adsorbent, human polyclonal IgG (1500 mg / 10 mL Gamma-Globulin Nichiyaku, produced by Nichiyaku) was selected, and the static binding capacity was measured. As a result, the static binding capacity was 83.9 mg/mL for the adsorbent. Further, the dynamic binding capacity was measured to find that the IgG adsorption capacity (5% dynamic binding capacity) at a linear rate of 300 cm/hour was 29.8 mg/mL for the adsorbent. Further, the IgG adsorption capacity (5% dynamic binding capacity) at a linear rate of 150 cm/hour was 36.0 mg/mL for the adsorbent.

Example 17: Production of adsorbent

An adsorbent on which protein A was immobilized was obtained in the same manner as in the Examples 15 and 16, except that the cellulose porous particle obtained in the Example 9 was used. The static binding capacity of the obtained adsorbent was measured; as a result, the static binding capacity was 85.9 mg/mL for the adsorbent. Further, the dynamic adsorption amount was measured to find that the IgG dynamic adsorption amount (5% dynamic binding capacity) at a linear rate of 300 cm/hour was 6.9 mg/mL for the adsorbent.

Example 18

An adsorbent on which protein A was immobilized was obtained in the same manner as in the Examples 15 and 16, except that the cellulose porous particle obtained in the Example 10 was used. The static binding capacity of the obtained adsorbent was measured; as a result, the static binding capacity was 49.3 mg/mL for the adsorbent. Further, the dynamic adsorption amount was measured to find that the IgG dynamic adsorption amount (5% dynamic binding capacity) at a linear rate of 300 cm/hour was 1.6 mg/mL for the adsorbent.

Example 19

An adsorbent on which protein A was immobilized was obtained in the same manner as in the Examples 15 and 16, except that the cellulose porous particle obtained in the Example 11 was used. The static binding capacity of the obtained adsorbent was measured; as a result, the static binding capacity was 79.3 mg/mL for the adsorbent. Further, the dynamic adsorption amount was measured to find that the IgG dynamic adsorption amount (5% dynamic binding capacity) at a linear rate of 300 cm/hour was 9.3 mg/mL for the adsorbent.

Example 20

An adsorbent on which protein A was immobilized was obtained in the same manner as in the Examples 15 and 16, except that the cellulose porous particle obtained in the Example 12 was used. The static binding capacity of the obtained adsorbent was measured; as a result, the static binding capacity was 58.9 mg/mL for the adsorbent. Further, the dynamic adsorption amount was measured to find that the IgG dynamic adsorption amount (5% dynamic binding capacity) at a linear rate of 300 cm/hour was 1.6 mg/mL for the adsorbent.

Example 21

An adsorbent on which protein A was immobilized was obtained in the same manner as in the Examples 15 and 16, except that the cellulose porous particle obtained in the Example 13 was used. The static binding capacity of the obtained adsorbent was measured; as a result, the static binding capacity was 83.9 mg/mL for the adsorbent. Further, the dynamic adsorption amount was measured to find that the IgG dynamic adsorption amount (5% dynamic binding capacity) at a linear rate of 300 cm/hour was 12.8 mg/mL for the adsorbent.

**Claims**

1. A method for producing a porous particle, comprising the steps of:

   preparing a solution containing cellulose and an ionic liquid;
   preparing a dispersion by dispersing the obtained cellulose solution into a liquid, wherein the liquid is not compatible with the ionic liquid; and
   coagulating the dispersion by bringing into contact with an alcohol or an alcohol aqueous solution in order to obtain the porous particle.

2. The production method according to claim 1, wherein water and/or an alcohol is added and an amount of the water and/or alcohol to be added is adjusted to be not less than 2 wt% and not more than 20 wt% relative to a total amount with the ionic liquid in the step for preparing the cellulose solution.

3. The production method according to claim 1 or 2, wherein a temperature of the cellulose solution is adjusted to be not less than 0°C and not more than 70°C.

4. The production method according to any one of claims 1 to 3, wherein the dispersion is brought into contact with the alcohol or the alcohol aqueous solution under the condition of not less than 10°C.

5. A porous particle, produced by the method according to any one of claims 1 to 4.

6. The porous particle according to claim 5, wherein a value of k is not more than -0.1 under the condition that the following formula (I) holds when the porous particle is packed in a column, gel phase distribution coefficients of multiple proteins having equal or higher molecular weight as compared to a target substance to be adsorbed are measured, and measured values are plotted with $K_{av}$ values on an abscissa and logarithm values of the molecular weights on an ordinate:

$$K_{av} = k \times \ln(MW) + b \quad (I)$$

wherein $K_{av}$ represents the gel phase distribution coefficient, MW represents the molecular weight of the protein, and b represents a constant.

7. The porous particle according to claim 6, wherein the $K_{av}$ value of the target substance to be adsorbed measured by using the column packed with the porous particle is not less than 0.3.

8. The porous particle according to claim 6, wherein the $K_{av}$ value of IgG measured by using the column packed with the porous particle is not less than 0.3.

9. The porous particle according to any one of claims 5 to 8, wherein an exclusion limit molecular weight is not less than $10^3$ and not more than $10^9$.

10. An adsorbent, comprising an affinity ligand and the porous particle produced by the method according to any one of claims 1 to 4 or the porous particle according to any one of claims 5 to 9, wherein the affinity ligand is immobilized on the porous particle.

11. The adsorbent according to claim 10, wherein an amount of the affinity ligand to be introduced is not less than 1 mg and not more than 1000 mg per ml of the porous particle.

12. The adsorbent according to claim 10 or 11, wherein an amount of cellulose to be contained is not less than 2 wt% and not more than 50 wt%.

13. The adsorbent according to any one of claims 10 to 12, wherein the porous particle is cross-linked.

14. The adsorbent according to any one of claims 10 to 13, wherein a compressive stress when the adsorbent is compressed by 10% is not less than 0.01 MPa and not more than 3 MPa.

15. The adsorbent according to any one of claims 10 to 14, wherein a volume average particle diameter is not less than 1 $\mu$m and not more than 2000 $\mu$m.

16. The adsorbent according to any one of claims 10 to 15, wherein the affinity ligand is protein A.

17. A method for purifying a protein, comprising the steps of:

packing the adsorbent according to any one of claims 10 to 16 into a column;
allowing a solution containing a crude protein to pass through the adsorbent packed in the column; and
allowing an eluent to pass through the column.

FIG.1

5.0kV x1.00k SE(M)                                         50.0um

50,0 μm

FIG.2

5.0kV x10.0k SE(M)                                         5.00um

5.00 μm

**FIG.3**

5.0kV x500 SE(M) 100um

100 μm

**FIG.4**

5.0kV x10.0K SE(M) 5.00um

5.00 μm

**FIG.5**

**FIG.6**

**FIG.7**

5.00 μm

**FIG.8**

100 μm

FIG.9

5.00 μ m

FIG.10

100 μ m

**FIG.11**

5.00 µm

**FIG.12**

300 µm

FIG.13

5,00 μm

FIG.14

20.0 μm

## FIG.15

5 0kV x30.0k SE(M)

1.00 μm

## FIG.16

5 0kV x1.00k SE(M)

50.0 μm

**FIG.17**

**FIG.18**

FIG.19

FIG.20

**FIG.21**

3.0kV x10.0k SE(M)　　　　　　　5.00μm

5.00 μm

**FIG.22**

2.00 μm

**FIG.23**

2.00 μm

**FIG.24**

2.00 μm

**FIG.25**

| | INTERNATIONAL SEARCH REPORT | International application No. |
|---|---|---|
| | | PCT/JP2011/070757 |

**A.  CLASSIFICATION OF SUBJECT MATTER**
*C08J9/28*(2006.01)i, *C08J3/16*(2006.01)i

According to International Patent Classification (IPC) or to both national classification and IPC

**B.  FIELDS SEARCHED**

Minimum documentation searched (classification system followed by classification symbols)
C08J9/28, C08J3/16

Documentation searched other than minimum documentation to the extent that such documents are included in the fields searched
Jitsuyo Shinan Koho        1922-1996   Jitsuyo Shinan Toroku Koho   1996-2011
Kokai Jitsuyo Shinan Koho   1971-2011   Toroku Jitsuyo Shinan Koho   1994-2011

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| A | WO 2008/067942 A1  (DOW WOLFF CELLULOSICS GMBH), 12 June 2008 (12.06.2008), claims; page 3, line 31 to page 4, line 4; page 5, lines 7 to 11 & US 2010/0272819 A1    & EP 2167568 A1 | 1-17 |
| A | WO 2009/021687 A1  (DOW WOLFF CELLULOSICS GMBH), 19 February 2009 (19.02.2009), claims; page 4, lines 15 to 30 & JP 2010-535868 A       & EP 2022801 A1 & CN 101772515 A | 1-17 |
| A | JP 2008-266401 A  (Nisshinbo Industries, Inc.), 06 November 2008 (06.11.2008), claims; paragraph [0038] (Family: none) | 1-17 |

☒  Further documents are listed in the continuation of Box C.    ☐  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 07 November, 2011 (07.11.11) | 22 November, 2011 (22.11.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

38

**INTERNATIONAL SEARCH REPORT**

| International application No. |
|---|
| PCT/JP2011/070757 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2009/101111 A1   (BASF SE),<br>20 August 2009 (20.08.2009),<br>claims; page 9, line 12 to page 10, line 8<br>& JP 2011-514401 A        & US 2010/0310853 A1<br>& CN 101970555 A        & KR 10-2010-0131977 A | 1-17 |
| A | CN 1470552 A   (UNIV DONGHUA),<br>28 January 2004 (28.01.2004),<br>abstract; examples<br>(Family: none) | 1-17 |
| A | WO 2009/036480 A1   (LENZING AG.),<br>26 March 2009 (26.03.2009),<br>claims<br>& JP 2010/539302 A        & US 2011/0009259 A1<br>& CN 101802016 A        & KR 10-2010-0074211 A | 1-17 |
| A | WO 2009/036479 A1   (LENZING AG.),<br>26 March 2009 (26.03.2009),<br>claims<br>& JP 2010-539301 A        & US 2011-0028608 A1<br>& CN 101821327 A        & KR 10-2010-0069694 A | 1-17 |
| A | JP 2010-195996 A   (Hitachi, Ltd.),<br>09 September 2010 (09.09.2010),<br>claims; paragraphs [0016] to [0020]<br>& US 2010/0222460 A1     & CN 101817986 A | 1-17 |
| A | WO 2008/084854 A1   (Asahi Kasei Fibers Corp.),<br>17 July 2008 (17.07.2008),<br>claims; paragraph [0031]; examples<br>& US 2010/0087552 A1     & EP 2103644 A1<br>& CN 101595168 A | 1-17 |
| A | JP 61-211342 A   (Asahi Chemical Industry Co.,<br>Ltd.),<br>19 September 1986 (19.09.1986),<br>claims; examples<br>(Family: none) | 1-17 |
| E,A | JP 2011-214003 A   (Kaneka Corp.),<br>27 October 2011 (27.10.2011),<br>claims; paragraphs [0030], [0031]<br>(Family: none) | 1-17 |

Form PCT/ISA/210 (continuation of second sheet) (July 2009)

**EP 2 626 381 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H01278534 A
- JP H11158202 A
- WO 2008146906 A
- JP 2008248466 A
- WO 2006004067 A

- US 5151350 B
- WO 2003080655 A
- JP 2006304633 A
- JP 2009071766 A

**Non-patent literature cited in the description**

- Annals of the New York Academy of Sciences. 2005, vol. 1051, 635-646
- *American Heart Journal,* 2006, vol. 152 (4
- **KASAI KENICHI et al.** Affinity chromatography. Tokyo Kagaku Dojin, 1991

- Handbook of Process Chromatography, A Guide to Optimization, Scale-Up and validation. Handbook of Process Chromatography, A Guide to Optimization, Scale-Up and validation. Academic Press, 1997, 368